# FASCICULE DE BREVET EUROPEEN

(11) **EP 4 619 462 B1**
(45) Date de publication et mention de la délivrance du brevet: **08.07.2026**
(21) Numéro de dépôt: 23804672.6
(22) Date de dépôt: 09.11.2023
(51) Int. Cl.: C08J 11/08, C07C 51/09, C07C 51/43, C07C 63/16

(54) **PROCEDE D'EXTRACTION ET DE TRANSFORMATION PAR ALCOOLYSE ET HYDROLYSE DE PHTALATES CONTENUS DANS DES PLASTIQUES PVC**
VERFAHREN ZUR EXTRAKTION UND TRANSFORMATION VON PHTHALATEN AUS PVC-KUNSTSTOFFEN MITTELS ALKOHOLYSE UND HYDROLYSE
METHOD FOR THE EXTRACTION AND TRANSFORMATION, BY ALCOHOLYSIS AND HYDROLYSIS, OF PHTHALATES CONTAINED IN PVC PLASTICS

(30) Priorité: 18.11.2022 FR 2211988
(43) Date de publication de la demande: 24.09.2025
(73) Titulaire: IFP Energies nouvelles, 92500 Rueil-Malmaison (FR)
(72) Inventeur: CHAUMONNOT, Alexandra, 92852 RUEIL-MALMAISON CEDEX (FR); MAJCHER, Jerome, 92852 RUEIL-MALMAISON CEDEX (FR); MEKKI-BERRADA, Adrien, 92852 RUEIL-MALMAISON CEDEX (FR); PASQUIER, David, 92852 RUEIL-MALMAISON CEDEX (FR)
(74) Mandataire: IFP Energies nouvelles
(86) Numéro de dépôt international: PCT/EP2023/081316
(87) Numéro de publication internationale: WO 2024/104887

(56) Documents cités:
- CN-B- 105 203 473
- JP-A- 2000 239 435
- JP-A- 2004 359 880
- JP-A- 2006 249 423
- JP-A- 2006 328 123
- JP-A- 2007 092 035
- SONG MATTHEW ET AL: "Recycling Phthalate Plasticisers from Soft PVC for the Synthesis of Phthalic Anhydride", RC CONFERENCE ON SCIENCE, ENGINEERING, AND TECHNOLOGY (10-11 AUGUST 2017, MATRIX @ BIOPOLIS, A*STAR, SINGAPORE), 1 June 2017 (2017-06-01), XP093051540

## Description

### Domaine technique

L'invention concerne le domaine du recyclage de plastiques à base de poly(chlorure de vinyle) (PVC), en particulier un procédé d'extraction et de transformation de phtalates, plastifiants entrant dans la composition de PVC, par réactions chimiques combinées d'alcoolyse et d'hydrolyse. Plus précisément, l'invention concerne un procédé de récupération de l'acide phtalique (AP) et d'un plastique PVC cible réutilisable à partir d'une charge de PVC contenant au moins un phtalate.

### Technique antérieure

Par définition, un plastique est un mélange constitué d'une matière polymérique de base et de nombreux additifs, l'ensemble étant susceptible d'être moulé ou façonné (en général à chaud et/ou sous pression), afin de conduire à un semi-produit ou à un objet. Une pratique communément admise est de dénommer ledit plastique par le nom du polymère qui le constitue. Ainsi, le plastique poly(chlorure de vinyle) (PVC) correspond en fait à l'association du polymère PVC, dénommé dans la suite de la description « résine PVC », avec divers additifs choisis en fonction des fonctionnalités requises pour ledit plastique. Lesdits additifs peuvent être des molécules ou macromolécules organiques ou bien des (nano)particules inorganiques et sont utilisés en fonction des propriétés qu'ils confèrent à la résine PVC : résistance à la chaleur, à la lumière ou à un stress mécanique (stabilisants), souplesse (plastifiants), facilité de mise en œuvre (lubrifiants), coloration (colorants/pigments), etc.

Plusieurs méthodes de recyclage des plastiques PVC existent : des méthodes dites conventionnelles par simple recyclage mécanique des plastiques, des méthodes impliquant des modifications de leur composition, voire des transformations chimiques des composés qui les constituent.

Depuis le milieu du XX^{ème} siècle, le recyclage du plastique PVC impliquant une action chimique a fait l'objet de nombreux travaux visant, dans une première étape, à solubiliser la résine PVC avec une proportion variable d'additifs puis, dans une deuxième étape, à récupérer ladite résine selon divers processus chimiques (précipitation, évaporation, etc.) en présence de tout ou partie des additifs solubles. Pour exemple, les brevets EP0945481, EP1268628 et EP2276801 visent à recycler respectivement des objets divers à base de PVC (tuyaux souples ou rigides, châssis de fenêtre, câbles, etc.) et spécifiquement des objets à base de PVC renforcés par des fibres (bâches, revêtements de sols, etc.) selon un procédé mettant en œuvre une première étape de dissolution dans un solvant organique de la résine PVC et des additifs solubles, suivie d'une deuxième étape de précipitation à la vapeur d'eau permettant la récupération de la résine et de la majorité des additifs.

Le maintien desdits additifs au sein du PVC ainsi récupéré pour être recyclé n'est toutefois pas toujours souhaitable. Par exemple, l'évolution au cours du temps des réglementations qui les concernent est impactante. Ainsi, certains plastifiants appartenant à la famille des phtalates, notamment largement utilisés pour formuler les PVC dits « souples » il y a une quarantaine d'années, ont été progressivement soumis à autorisation en Europe sur la base du règlement REACH qui vise, depuis fin 2006, à sécuriser la fabrication et l'utilisation des substances chimiques dans l'industrie européenne et, finalement, progressivement exclus des additifs utilisables. C'est notamment le cas pour la liste non exhaustive des phtalates suivants : phtalate de dibutyle (DBP), phtalate de dioctyle ou de diéthylhexyle (DOP ou DEHP), phtalate de benzyle et butyle (BBP), phtalate de diisobutyle (DIBP), phtalate de dipentyle (DPP), phtalate de diisopentyle, phtalate de n-pentyle et isopentyle, phtalate de dihexyle, etc.

Ces nouvelles réglementations conduisent aujourd'hui à l'interdiction de la présence de tels composés dans des matières premières recyclées (MPR). En tenant compte de la durée de vie souvent très longue des objets à base de PVC (plusieurs dizaines d'années), les objets à base de PVC formulés antérieurement à fin 2006 et aujourd'hui en fin de vie ne peuvent être recyclés via des méthodes de régénération conduisant au maintien de ces additifs interdits, que lesdites méthodes soient conventionnelles, comme les procédés de recyclage mécanique, ou non, comme les exemples de procédé par dissolution/précipitation cités ci-dessus.

Par ailleurs, les plastifiants phtalates utilisés aujourd'hui en Europe (phtalates dits REACH compatibles) et dans le reste du monde représentent des additifs à forte valeur ajoutée qui ne sont pas en l'état valorisés lors de leur maintien dans la matière première recyclée PVC. En effet, ce sont des produits coûteux, présents dans des proportions non négligeables dans les formulations initiales des PVC (plusieurs dizaines de pourcents) et ne permettant pas de conférer directement à la MPR PVC les propriétés de souplesse *ad hoc.* L'apport de plastifiants « frais » en quantité conséquente est alors indispensable à la réemployabilité de la matière PVC recyclée.

L'extraction des additifs de type phtalate d'objets à base de PVC pour élimination ou valorisation représente donc un enjeu majeur pour une recyclabilité optimisée du PVC.

Plusieurs procédés mettant en jeu une étape de dissolution de la résine PVC ont été adaptés pour permettre cette extraction. Par exemple, les brevets EP1311599 et JP2007191586 proposent tous deux une première étape de dissolution de la résine PVC et au moins des additifs de type phtalates par un premier solvant organique, suivie d'une deuxième étape d'extraction liquide-liquide des phtalates de la solution obtenue précédemment, via l'emploi d'un deuxième solvant organique différent du premier. Le brevet JP2007092035 divulgue un autre exemple de mise en œuvre possible avec une dissolution de la résine PVC et au moins des additifs de type phtalates via l'utilisation d'un solvant en conditions supercritiques et la récupération desdits phtalates dans ce même solvant après « rupture » desdites conditions supercritiques.

L'élimination ou la valorisation d'additifs de type phtalates d'un plastique PVC peut également être mise en œuvre sans passer par une étape préliminaire de dissolution dudit plastique, via notamment une extraction directe desdits phtalates de la matrice polymérique solide par un solvant organique adéquat, comme cela est parfaitement répertorié dans la publication de Ügdüler et al., 2020, « Challenge and opportunities of solvent-based additive extraction methods for plastic recycling », Waste Management, 104, 148-182. L'enjeu réside alors dans l'optimisation des conditions d'extraction (nature du solvant, temps de contact, température, pression, etc.) pour atteindre les meilleurs rendements possibles en phtalates extraits. Bien que cette méthodologie d'élimination des phtalates de plastiques PVC soit fréquemment utilisée, notamment pour détecter et quantifier analytiquement ces additifs spécifiques dans lesdits plastiques, à notre connaissance, aucun procédé de régénération d'objets à base de PVC ne fait intervenir cette technique.

Bien que critique pour assurer un recyclage efficient des plastiques PVC et obtenir un PVC recyclé réemployable, l'extraction des plastifiants de type phtalate n'est pas suffisante pour assurer la viabilité économique d'un procédé de régénération d'objets à base de PVC. La principale raison fréquemment avancée est la difficulté à trouver un équilibre économiquement viable entre le coût des opérations unitaires mises en œuvre dans ledit procédé de régénération et le coût de revente (équivalent à la valeur ajoutée) des produits obtenus. Ces derniers sont constitués de la matière recyclée à base de PVC exempte de phtalates, naturellement valorisable, et desdits phtalates extraits qui, eux, le sont peu. En effet, tout procédé de régénération mettant en œuvre une étape d'extraction des phtalates d'objets à base de PVC va conduire à la récupération d'un mélange de phtalates, ce dernier pouvant comprendre des phtalates non « REACH compatibles ». La valorisation desdits phtalates non REACH compatibles est bien sûr exclue et ces derniers devront être traités en tant que déchets spécifiques générant des coûts supplémentaires. La valorisation des phtalates REACH compatibles, intéressante en tant que telle, est en fait délicate car impliquant des étapes de séparation/purification techniquement complexes et coûteuses.

Par le passé, quelques travaux ont porté sur la mise en contact de plastiques PVC contenant des phtalates avec des solutions aqueuses basiques fortement concentrées (essentiellement NaOH) pour transformer lesdits phtalates et extraire le(s) produit(s) résultant(s) : un sel de l'acide phtalique et d'éventuels produits de dégradation en fonction des conditions opératoires associées. Cette réaction chimique a été opérée conjointement ou en amont d'une étape de déchloration du PVC, permettant alors d'obtenir un résidu non chloré et majoritairement exempt de phtalates pour permettre sa valorisation énergétique. La réalisation d'une telle étape en amont à la déchloration, et assistée par des hautes fréquences ou micro-ondes, présente l'avantage de récupérer un sel d'acide phtalique valorisable, comme cela a été étudié dans les documents suivants : brevet JP3929352 ; F. Osada et al., 2010, « Deplasticization and dechlorination of flexible polyvinyl chloride in NaOH solution by microwave heating », J. Mater. Cycles Waste Manag., 2010, 12, 245 ; S. M. Shin et al., « Elution Behavior of Additive Agent from Flexible PVC », 2001, Chawon Rissaikuring, 10, 6, 3. Cette mise en œuvre présente néanmoins les inconvénients majeurs suivants : elle nécessite l'emploi de bases fortement concentrées, elle conduit à l'obtention non pas de l'acide phtalique mais de son sel associé et l'extraction des phtalates n'est pas optimisée et ne répond pas à la réglementation REACH applicable depuis 2006 pour la récupération d'un composé valorisable comme matière première recyclée.

D'autres exemples de méthodes pour la récupération de phtalates à partir d'une charge PVC contenant au moins un phtalate sont décrits dans les documents CN 105 203 473 B, JP 2004 359880 A et JP 2006 249423 A.

Des exemples de méthodes permettant de récupérer l'acide phtalique à partir d'une charge PVC contenant au moins un phtalate sont décrits dans l'article de M. Song et al., «Recycling Phthalate Plasticisers from Soft PVC for the Synthesis of Phthalic Anhydride», IRC Conference on Science, Engineering, and Technology, Singapour 2017, dans JP 2000 239435 A et JP 2006 328123 A.

### Résumé de l'invention

La présente invention a pour objectif de surmonter, au moins en partie, les problèmes de l'art antérieur, et vise en particulier à fournir un procédé de régénération d'objets à base de PVC permettant le traitement de tout type de charge de PVC contenant des phtalates et leur transformation en deux produits d'intérêt susceptibles d'être valorisés comme matières premières : l'acide phtalique et un plastique PVC recyclable exempt de phtalates, notamment de phtalates indésirables, typiquement ceux soumis à autorisation par la réglementation européenne REACH.

L'acide phtalique est notamment utilisé pour fabriquer les phtalates, qui sont des dérivés de l'acide phtalique. L'acide phtalique peut être utilisé comme matière première pour la fabrication d'autres produits chimiques, dans des domaines différents de celui de la formulation de plastiques, par exemple pour fabriquer des colorants, parfums, édulcorants comme la saccharine, etc.

Ainsi, pour atteindre au moins l'un des objectifs susvisés, parmi d'autres, la présente invention propose, selon un premier aspect, un procédé de récupération de l'acide phtalique (AP) et d'un plastique PVC cible réutilisable à partir d'une charge de PVC contenant au moins un phtalate en deux temps :
- une première série d'étapes : les étapes a) à d), et les étapes optionnelles e), f1), f2), permettant d'obtenir le plastique PVC cible et, notamment par la mise en œuvre d'une réaction d'alcoolyse, au moins un produit intermédiaire de type dialkylphtalate, qui est aisément séparable ,
- une deuxième série d'étapes : les étapes g) et h) mettant notamment en œuvre une réaction d'hydrolyse du dialkylphtalate généré lors de la première série, et permettant d'obtenir *in fine* au moins un flux d'acide phtalique solide afin de récupérer l'acide phtalique.

Le procédé selon l'invention permet ainsi de produire une poudre d'acide phtalique de bonne pureté à partir d'une charge PVC, typiquement un déchet PVC, sans consommation stœchiométrique de base ni d'acide.

Plus précisément, la première série d'étapes comprend les étapes a) à d) suivantes, qui sont également décrites dans la demande de brevet français déposée sous le numéro 21/05.299 :
a) une extraction solide-liquide d'une charge de PVC sous forme de particules par mise en contact des particules de ladite charge avec un solvant comportant au moins un alcool de formule CₙH₂ₙ₊₁OH, n entier positif inférieur à 4 ou supérieur à 8, pour produire une phase liquide enrichie en ledit phtalate et une phase solide comportant du plastique PVC appauvri en ledit phtalate ;
b) la transformation chimique dudit phtalate de la phase liquide en dialkylphtalate de formule C₆H₄(COOCₙH₂ₙ₊₁)₂ par transestérification au moyen dudit alcool (alcoolyse) pour enrichir ladite phase liquide en ledit dialkylphtalate ;
c) une séparation solide-liquide entre ladite phase solide et ladite phase liquide pour produire au moins un flux solide comportant le plastique PVC appauvri en ledit phtalate afin de récupérer le plastique PVC cible ;
d) une séparation (gaz-liquide ou liquide-liquide) de la phase liquide, pour produire au moins un premier effluent liquide comportant le dialkylphtalate et un deuxième effluent liquide comprenant le solvant.

La première série d'étapes peut également comprendre les étapes e), f₁), f₂) suivantes, également décrites dans la demande de brevet français déposée sous le numéro 21/05.299 :
e) une purification optionnelle du premier effluent liquide obtenu à l'étape d) comprenant ledit dialkylphtalate, du phtalate partiellement converti et/ou non converti à l'étape b) et éventuellement des impuretés solubles, pour produire un produit liquide consistant essentiellement en ledit dialkylphtalate, et un résidu liquide comprenant dudit phtalate partiellement converti et/ou non converti à l'étape b) et éventuellement lesdites impuretés solubles ;
f) une étape optionnelle supplémentaire f₁) et/ou étape optionnelle supplémentaire f₂) de transformation chimique par transestérification dudit phtalate non converti et/ou partiellement à l'étape b), en dialkylphtalate de formule C₆H₄(COOCₙH₂ₙ₊₁)₂ au moyen dudit alcool, ladite étape f₁) étant réalisée entre les étapes c) et d) par envoi de ladite phase liquide obtenue à l'issue de l'ensemble des étapes a), b) et c) dans un premier réacteur de transestérification supplémentaire pour produire un deuxième flux liquide enrichi en ledit dialkylphtalate de formule C₆H₄(COOCₙH₂ₙ₊₁)₂, le deuxième flux liquide étant envoyé à l'étape d), et ladite étape f₂) étant réalisée successivement à l'étape e) par envoi dudit résidu liquide dans un deuxième réacteur de transestérification supplémentaire pour produire un troisième flux liquide enrichi en ledit dialkylphtalate de formule C₆H₄(COOCₙH₂ₙ₊₁)₂, le troisième flux liquide étant renvoyé à l'étape d) ;

La deuxième série d'étapes du procédé selon l'invention comporte les étapes g) et h) suivantes :
g) une transformation chimique du dialkylphtalate obtenu lors de l'étape d) ou lors de l'étape optionnelle e) en acide phtalique de formule C₆H₄(COOH)₂ par hydrolyse en présence d'eau, pour produire au moins un effluent comportant une phase aqueuse enrichie en ledit acide phtalique ;
h) une étape de séparation dudit effluent obtenu à l'étape g) pour produire au moins un flux solide d'acide phtalique (acide phtalique à l'état solide, e.g. sous forme de poudre, en particulier formant des paillettes ou aiguilles).

Un intérêt de la présente invention réside dans la capacité du procédé, grâce à une réaction chimique de transestérification (alcoolyse), à transformer un mélange de phtalates initialement piégés dans des matrices polymériques de divers objets à base de plastique PVC, quelle que soit la composition dudit mélange (c'est-à-dire quelle que soit la nature et l'origine des différents phtalates) et malgré la présence possible de nombreux autres additifs, en un produit unique qui est un DAP, qui peut alors lui-même être isolé, puis chimiquement transformé par réaction d'hydrolyse en acide phtalique. Ledit acide phtalique est un produit précurseur de multiples phtalates REACH compatibles, encore très largement utilisés dans de nombreux domaines comme celui de la plasturgie.

L'obtention de l'acide phtalique par l'intermédiaire d'un unique dialkylphtalate formé lors des étapes a) à d), et optionnellement e) et f₁) et/ou f₂), permet en outre d'assurer un degré de pureté accru par rapport à d'autres procédés conventionnels de production d'acide phtalique. En effet l'obtention du seul produit dialkylphtalate à partir du mélange de phtalates permet d'en faciliter la séparation. Cette séparation permet d'isoler ce dialkylphtalate et de réaliser ensuite la réaction d'hydrolyse sur un réactif déjà exempt de nombreux composés indésirables présents dans le PVC ou dans l'étape d'extraction (additifs, alcools issus de l'alcoolyse, PVC, produits de dégradation). Ceci garantit la génération d'acide phtalique par hydrolyse avec un degré de pureté important tout en limitant le nombre d'étapes unitaires associées aux opérations de séparation/purification et donc de limiter les coûts. Par ailleurs, la réaction d'hydrolyse pour générer l'acide phtalique, par opposition à une saponification, permet de s'affranchir d'une consommation de base (telle NaOH) en quantité stœchiométrique et d'acide. Le procédé selon l'invention s'inscrit ainsi parfaitement dans une stratégie d'économie circulaire.

Selon une première variante, les étapes a) et b) sont mises en œuvre au sein d'une même opération unitaire, produisant un flux comportant la phase aqueuse comportant de l'acide phtalique et la phase solide comportant du plastique PVC appauvri en ledit phtalate.

Selon une deuxième variante alternative à la première variante, les étapes a) et b) font l'objet de deux opérations unitaires distinctes, l'étape a) produisant un flux comportant ladite phase liquide et ladite phase solide envoyé à l'étape c) de séparation solide-liquide réalisée entre les étapes a) et b), l'étape c) produisant ledit flux comportant le plastique PVC appauvri en ledit phtalate et un premier flux liquide comportant ladite phase liquide envoyée à l'étape b).Selon un ou plusieurs modes de réalisation, l'hydrolyse à l'étape g) est réalisée en présence d'un catalyseur d'hydrolyse acide, de préférence un catalyseur homogène acide choisi dans la liste constituée par les catalyseurs acides de Brônsted minéraux, de préférence l'acide chlorhydrique, l'acide sulfurique, l'acide phosphorique, les catalyseurs acides de Brônsted organiques, de préférence l'acide *p*-toluènesulfonique, et les catalyseurs acides de Lewis, de préférence AlF₃, ou un catalyseur hétérogène acide choisi dans la liste constituée par les alumines, les alumines chlorées, les alumines fluorées, les aluminosilicates mésoporeux, les zéolithes et leurs mélanges avec d'autres oxydes, les résines échangeuses d'ions (H+), de préférence les résines sulfoniques.

Selon un ou plusieurs modes de réalisation, l'hydrolyse à l'étape g) est réalisée à une température comprise entre la température ambiante et 150°C, de préférence comprise entre 40°C et 130°C, à une pression comprise entre la pression atmosphérique et 5,0 MPa, de préférence comprise entre la pression atmosphérique et 2,0 MPa, et pendant une durée comprise entre 1 minute et 10 heures, de préférence comprise entre 10 minutes et 4 heures.

Selon un ou plusieurs modes de réalisation, l'hydrolyse à l'étape g) est réalisée de sorte que le rapport molaire entre la quantité d'eau et la quantité dudit au moins un phtalate à transformer extrait à l'étape a) soit compris entre 100 et 9000.

Selon un ou plusieurs modes de réalisation, l'étape h) comporte changement de phase de l'acide phtalique de l'état dissous dans ladite phase aqueuse à un état solide et une séparation solide-liquide pour produire ledit flux solide d'acide phtalique et au moins un flux liquide aqueux.

Selon un ou plusieurs modes de réalisation, le premier effluent liquide à l'étape d) ou le produit liquide à l'étape optionnelle e) consiste essentiellement en ledit dialkylphtalate.

Selon un ou plusieurs modes de réalisation, le flux solide comportant le plastique PVC appauvri en phtalates est recyclé au moins en partie à l'étape a).

Selon un ou plusieurs modes de réalisation, le deuxième effluent liquide comprenant au moins ledit solvant issu de l'étape d) est recyclé, au moins en partie, à l'étape a) et/ou l'étape b).

Selon un ou plusieurs modes de réalisation, l'alcool est choisi dans la liste constituée par le méthanol, l'éthanol, le n-propanol, l'i-propanol, et de préférence le méthanol, ou dans la liste constituée par le nonanol, linéaire ou ramifié, le décanol, linéaire ou ramifié, le undécanol, linéaire ou ramifié, le dodécanol, linéaire ou ramifié, et de préférence le nonanol ou le décanol.

Selon un ou plusieurs modes de réalisation, le solvant comprend en outre un co-solvant organique, de préférence ledit co-solvant organique étant choisi parmi un ester dérivé dudit alcool et étant de formule R'COOCₙH₂ₙ+1, R' étant un groupement alkyle, de préférence comprenant entre 1 et 3 atomes de carbone, et un éther, de préférence ledit co-solvant organique est choisi dans le groupe constitué par l'acétate de méthyle, le propanoate de méthyle, et le cyclopentylméthyléther, et ledit co-solvant organique étant additionné audit alcool de sorte que le ratio massique entre ledit co-solvant organique et ledit alcool soit compris entre 0,01 et 4.

Selon un ou plusieurs modes de réalisation, le co-solvant organique est choisi dans le groupe constitué par l'acétate de méthyle, le propanoate de méthyle, et le cyclopentylméthyléther.

Selon un ou plusieurs modes de réalisation, ledit alcool est du méthanol, ledit dialkylphtalate est le diméthylphtalate, et ledit solvant comprend de préférence du propanoate de méthyle de sorte que le ratio massique entre ledit propanoate de méthyle et ledit alcool soit compris entre 0,01 et 4.

Selon un ou plusieurs modes de réalisation, lequel la transformation chimique réalisée par transestérification à l'étape b), et éventuellement à l'étape f₁) et/ou f₂), est opérée :
- à une température comprise entre la température ambiante et 200°C, de préférence comprise entre 40°C et 180°C,
   à une pression comprise entre la pression atmosphérique et 11,0 MPa, de préférence comprise entre la pression atmosphérique et 5,0 MPa,
- pendant une durée comprise entre 1 minute et 10 heures, de préférence comprise entre 10 minutes et 4 heures,
- avec un rapport molaire entre la quantité dudit alcool du solvant et la quantité dudit phtalate à extraire ou à transformer est compris entre 2 et 250, de préférence compris entre 4 et 90, et
- en présence d'un catalyseur de transestérification, de préférence choisi dans la liste constituée par les catalyseurs homogènes basiques, ou acides de Brônsted minéraux ou organiques, ou acides de Lewis, et les catalyseurs hétérogènes formés par des oxydes de métaux alcalino-terreux, ou des carbonates ou hydrogéno-carbonates de métaux alcalins et/ou alcalino-terreux, ou des métaux alcalins supportés sur alumines ou zéolites, ou des oxydes de zinc et leurs mélanges avec d'autres oxydes, ou des résines échangeuses d'ions.

Selon un ou plusieurs modes de réalisation, ledit au moins un phtalate de ladite charge de PVC est un phtalate de formule brute C₆H₄(COOR₁)(COOR₂) dont les groupements esters sont en position ortho du noyau benzénique, R₁ ou R₂ étant choisis indépendamment parmi l'un des éléments du groupe constitué par une chaîne alkyle, linéaire ou ramifiée ou cyclique, une chaîne alkoxyalkyle, linéaire ou ramifiée, ou une chaîne aryle ou alkylaryle, R₁ et/ou R₂ comprenant de préférence entre 1 et 20 atomes de carbone, voire entre 1 et 15 atomes de carbone.

Selon un ou plusieurs modes de réalisation, ledit plastique PVC cible est sensiblement exempt dudit phtalate, et de préférence comprend moins de 0,1% massique au total de phtalates choisis dans la liste constituée par le phtalate de dibutyle, phtalate de dioctyle ou de diéthylhexyl, phtalate de benzyle et butyle, phtalate de dibutyle, phtalate de diisobutyle, phtalate de dipentyle, phtalate de diisopentyle, phtalate de n-pentyle et isopentyle, phtalate de dihexyle, phtalate de bis(2-méthoxyéthyle), et leurs mélanges.

Selon un deuxième aspect, la présente invention porte sur un procédé de recyclage d'un objet à base de PVC contenant au moins un phtalate comportant :
- le conditionnement dudit objet à base de PVC comprenant au moins un broyage ou un déchiquetage dudit objet à base de PVC pour former une charge de PVC sous forme de particules ;
- la récupération d'acide phtalique et d'un plastique PVC cible réutilisable à partir de ladite charge de PVC sous forme de particules selon le premier aspect l'invention.

La présente invention concerne également, selon un troisième aspect, un procédé de fabrication d'un objet à base de PVC souple comportant un plastique PVC recyclé et/ou un phtalate fabriqué à partir d'acide phtalique récupéré par le procédé selon le premier aspect de l'invention.

D'autres objets et avantages de l'invention apparaîtront à la lecture de la description qui suit, d'exemples de réalisation particuliers de l'invention, donnés à titre d'exemples non limitatifs, la description étant faite en référence aux figures annexées décrites ci-après.

### Liste des figures

La figure 1 est un schéma illustrant une partie du procédé (première série d'étapes) selon un mode de réalisation de l'invention comportant les étapes a), b), c) et d).
La figure 2 est un schéma d'une partie du procédé (première série d'étapes) selon un autre mode de réalisation comportant les étapes a), b), c) et d), avec à l'étape d) une séparation entre le DAP, le solvant, des sous-produits de type alcool obtenus à l'étape b) et les phtalates partiellement convertis et/ou non convertis à l'étape b) éventuellement en mélange avec des impuretés solubles.
La figure 3 est un schéma illustrant une partie du procédé (première série d'étapes) selon les modes de réalisation illustrés à la figure 1 ou à la figure 2, comportant les étapes a), b), c), d), et illustrant la mise en œuvre d'autres étapes optionnelles de transestérification (f₁) et de recyclage de divers flux.
La figure 4 est un schéma illustrant une partie du procédé (première série d'étapes) selon un autre mode de réalisation de l'invention comportant les étapes a), b), c), d) ainsi qu'une étape de purification e) d'un premier effluent obtenu à l'étape d) comprenant le DAP.
La figure 5 est un schéma illustrant une partie du procédé (première série d'étapes) selon le mode de réalisation illustré à la figure 4, et illustrant la mise en œuvre d'autres étapes optionnelles de transestérification (f₁ ; f₂) et de recyclage de divers flux.
La figure 6 est un schéma illustrant une partie du procédé (première série d'étapes) selon un mode de réalisation préféré de l'invention, comportant une mise en œuvre au sein d'une même opération unitaire des étapes a) et b) (première variante du procédé selon l'invention), une étape de purification e) d'un premier effluent obtenu à l'étape d) comprenant le DAP et une étape supplémentaire de transestérification f₂) du résidu issu de l'étape e).
La figure 7 est un schéma illustrant une partie du procédé (première série d'étapes) selon un autre mode de réalisation de l'invention comportant les étapes a), b), c), d), dans lequel les étapes a) et b) font l'objet de deux opérations unitaires distinctes (deuxième variante du procédé selon l'invention), et dans lequel l'étape c) est réalisée entre les étapes a) et b).
La figure 8 est un schéma illustrant une partie du procédé (première série d'étapes) tel qu'illustré à la figure 7, selon un mode de réalisation préféré comportant une étape de purification e) d'un premier effluent obtenu à l'étape d) comprenant le DAP et une étape supplémentaire de transestérification f₂) du résidu issu de l'étape e).
La figure 9 est un schéma illustrant de manière générique une partie du procédé (deuxième série d'étapes) comportant les étapes g) et h), et de manière optionnelle l'utilisation d'un solvant d'extraction non miscible à l'eau à l'étape de séparation h).
La figure 10 est un schéma illustrant une partie du procédé (deuxième série d'étapes) selon un autre mode de réalisation dans lequel l'étape de séparation h) comporte deux sous-étapes h1) et h2).
La figure 11 est un schéma illustrant une partie du procédé (deuxième série d'étapes) selon un autre mode de réalisation dans lequel l'étape de séparation h) comporte deux sous-étapes h3) et h4).
La figure 12 est un schéma illustrant une partie du procédé (deuxième série d'étapes) selon un autre mode de réalisation dans lequel l'étape de séparation h) comporte trois sous-étapes h5), h6) et h2).
La figure 13 est un schéma illustrant une partie du procédé (deuxième série d'étapes) selon un autre mode de réalisation dans lequel l'étape de séparation h) comporte trois sous-étapes h7), h8) et h4).

Sur les figures, les mêmes références désignent des éléments identiques ou analogues.

### Description des modes de réalisation

### Terminologie

Certaines définitions sont données ci-dessous, bien que plus de détails sur les objets définis ci-après puissent être donnés plus loin dans la description.

On entend par objet à base de PVC, un objet, en général de consommation, qui comprend, et de préférence est constitué de, au moins un plastique PVC.

On entend par plastique poly(chlorure de vinyle), dénommé encore plastique PVC ou simplement PVC, la combinaison d'un polymère PVC, encore appelé résine PVC, avec divers additifs choisis en fonction des fonctionnalités requises pour le plastique PVC, elles-mêmes choisies en fonction des applications visées.

Ledit polymère PVC est issu de la polymérisation radicalaire du chlorure de vinyle (VCM), monomère lui-même obtenu à partir de chlore et d'éthylène. En fonction de la mise en œuvre de ladite polymérisation, quatre familles de résines PVC sont utilisables : 1) les résines PVC suspension ou PVC-S (polymérisation en suspension du VCM), 2) les résines PVC émulsion ou PVC « pâtes » (polymérisation en émulsion), 3) les résines PVC masse ou PVC-M (polymérisation en masse) et 4) les résines PVC surchlorées ou PVC-C, obtenues par sur-chloration en post-traitement des résines précédentes.

Lesdits additifs entrant dans la composition d'un plastique PVC peuvent être des molécules ou macromolécules organiques ou bien des (nano)particules inorganiques et sont utilisés en fonction des propriétés qu'ils confèrent à la résine PVC : résistance à la chaleur, à la lumière ou à un stress mécanique (stabilisants), souplesse (plastifiants), facilité de mise en œuvre (lubrifiants), coloration (colorants/pigments), etc.

On entend par phtalates, le groupe de produits chimiques formé par les diesters carboxyliques de l'acide phtalique. Ils sont composés d'un noyau benzénique et de deux groupements esters carboxyliques placés en position ortho du noyau benzénique. Ils peuvent être décrits à l'aide de la formule suivante : 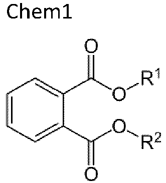 ou encore par la formule brute C₆H₄(COOR₁)(COOR₂), où R₁ et R₂ sont choisis indépendamment parmi l'un des éléments du groupe constitué par une chaîne alkyle, linéaire, ramifiée ou cyclique, une chaîne alkoxyalkyle, linéaire ou ramifiée, ou une chaîne aryle ou alkylaryle, ladite chaîne alkyle, alkoxyalkyle, aryle ou alkylaryle pouvant typiquement comporter entre 1 et 20 atomes de carbone, voire comporter entre 1 et 15 atomes de carbone. Par exemple, R₁ et/ou R₂ peuvent être choisis indépendamment parmi les groupes éthyle, n-butyle, iso-butyle, n-pentyle, iso-pentyle, n-hexyle, n-octyle, n-nonyle, iso-nonyle, n-décyle, iso-décyle, méthoxyéthyle, benzyle.

Les phtalates sont couramment utilisés comme plastifiants des matières plastiques et en particulier comme plastifiants des plastiques du type PVC, notamment pour les rendre souples.

Dans la présente description, le terme « dialkylphtalate » (DAP) désigne le produit de formule brute C₆H₄(COOCₙH₂ₙ₊₁)₂ résultant de la réaction de transestérification d'au moins un plastifiant de type phtalate (et en particulier de formule brute C₆H₄(COOR₁)(COOR₂), comme décrite ci-dessus) présent dans des objets à base de PVC avec un alcool de formule brute CₙH₂ₙ₊₁OH, n < 4 ou n > 8. Le diméthylphtalate est un exemple préféré de DAP.

Dans la présente description, la définition dudit alcool de formule brute CₙH₂ₙ₊₁OH, n < 4 ou n > 8, peut comprendre également sa base conjuguée de formule brute de CₙH₂ₙ₊₁O⁻, avec n < 4 ou n > 8, le contre-ion cationique, y compris de nature métallique, assurant l'électronégativité de ladite base conjuguée étant bien connu de l'Homme du métier. Ladite base conjuguée est également appelée forme « alcoolate » ou « alcoxyde » dudit alcool.

On entend par « sous-produit(s) de type alcool issus de la réaction d'alcoolyse » (AL_{A}), le(s) sous-produit(s) de formule R₁OH ou R₂OH résultant de la réaction de transestérification entre au moins un plastifiant de type phtalate présent dans des objets à base de PVC avec l'alcool de formule brute CₙH₂ₙ₊₁OH, n < 4 ou n > 8. R₁ ou R₂ sont définis de manière identique à R₁ et R₂ des phtalates. Comme précédemment, la définition dudit sous-produit de type alcool de formule R₁OH ou R₂OH peut comprendre également sa base conjuguée de formule brute R₁O⁻ ou R₂O⁻.

On entend par « alkylphtalate intermédiaire issus de la réaction d'alcoolyse » (API_{A}) ou « phtalate partiellement converti après alcoolyse », le sous-produit de formule brute C₆H₄(COOR₁)(COOCₙH₂ₙ₊₁) ou C₆H₄(COOR₂)(COOCₙH₂ₙ₊₁) résultant de la réaction de transestérification incomplète d'au moins un plastifiant de type phtalate (et en particulier de formule brute C₆H₄(COOR₁)(COOR₂), comme décrite ci-dessus) présent dans des objets à base de PVC avec un alcool de formule brute CₙH₂ₙ₊₁OH, n < 4 ou n > 8. R₁ ou R₂ sont définis de manière identique à R₁ et R₂ des phtalates.

Dans la présente description, le terme « acide phtalique » (AP), également connu sous le nom acide benzène-1,2-dicarboxylique ou acide o-phtalique, désigne le produit de formule brute C₆H₄(COOH)₂ résultant de la réaction d'hydrolyse entre le DAP tel que décrit ci-dessus et de l'eau (H₂O).

On entend par « sous-produit(s) de type alcool issus de la réaction d'hydrolyse » (AL_{H}), le sous-produit de formule CₙH₂ₙ₊₁OH, avec n < 4 ou n > 8, résultant de la réaction d'hydrolyse entre le DAP tel que décrit ci-dessus et H₂O.

On entend par « alkylphtalate intermédiaire issus de la réaction d'hydrolyse » (API_{H}) ou « dialkylphtalate partiellement converti après alcoolyse », le sous-produit de formule brute C₆H₄(COOH)(COOCₙH₂ₙ₊₁), avec n < 4 ou n > 8, résultant de la réaction d'hydrolyse incomplète entre le DAP tel que décrit ci-dessus et H₂O.

On entend par « plastique PVC cible réutilisable », un « PVC exempt de phtalates », c'est-à-dire le solide comprenant au moins la résine PVC additionnée d'au moins un des additifs présents initialement dans le plastique PVC de la charge de PVC traitée selon l'invention, et duquel les phtalates ont été extraits et transformés sous la forme d'au moins l'AP selon l'invention. Les termes « exempt de phtalates » signifient en particulier que le PVC solide obtenu comme produit du procédé selon l'invention contient, au total, moins de 0,1% en masse de phtalates soumis à autorisation par la réglementation REACH en Europe (annexe XIV du règlement (CE) N°1907/2006 du parlement européen et du conseil du 18 décembre 2006), en particulier moins de 0,1% en masse de phtalates choisis dans la liste constituée par les phtalates suivants : phtalate de dibutyle (DBP), phtalate de dioctyle ou de diéthylhexyl (DOP ou DEHP), phtalate de benzyle et butyle (BBP), phtalate de dibutyle (DBP), phtalate de diisobutyle (DIBP), phtalate de dipentyle (DPP), phtalate de diisopentyle, phtalate de n-pentyle et isopentyle, phtalate de dihexyle, phtalate de bis(2-méthoxyéthyle), seuls ou en mélange.

Dans la présente description, l'alcool de formule brute CₙH₂ₙ₊₁OH, avec n < 4 ou n > 8, éventuellement additionné d'au moins un co-solvant organique, est également dénommé « solvant ».

Dans la présente description, le solvant éventuellement utilisé lors de l'étape h) de séparation produisant un flux solide d'acide phtalique est spécifiquement dénommé « solvant de séparation » afin d'éviter toute confusion avec l'alcool de formule brute CₙH₂ₙ₊₁OH, avec n < 4 ou n > 8, éventuellement additionné d'au moins un co-solvant organique.

Dans la présente description, l'expression « supérieur à... » est entendue comme strictement supérieur, et symbolisée par le signe « > », et l'expression « inférieur à » comme strictement inférieur, et symbolisée par le signe « < ».

Dans la présente description, l'indice « n » des formules chimiques citées est un entier positif (c'est-à-dire strictement supérieur à zéro). Selon l'invention, n est inférieur à 4 ou supérieur à 8, et de préférence inférieur ou égal à 20, voire inférieur ou égal à 15.

Dans la présente description, on entend par température ambiante (Tamb) une température typiquement de 20°C ± 5°C, et par pression atmosphérique une pression de 0,101325 MPa.

Dans la présente description, le terme « comprendre » est synonyme de (signifie la même chose que) « comporter », « inclure » et « contenir », et est inclusif ou ouvert et n'exclut pas d'autres éléments qui ne seraient pas mentionnés. Il est entendu que le terme « comprendre » inclut le terme exclusif et fermé « consister ».

Dans la présente description, l'expression « compris entre ... et ... » signifie que les valeurs limites de l'intervalle sont incluses dans la gamme de valeurs décrite, sauf spécifié autrement.

Dans la présente description, on entend qu'un flux « consiste essentiellement » en un composé, un flux comprenant au moins 95% massique dudit composé, de préférence au moins 98% massique, et plus préférentiellement au moins 99% massique dudit composé.

Dans la présente description, les différentes plages de paramètres pour une étape donnée telles que les plages de pression et les plages température peuvent être utilisées seule ou en combinaison. Par exemple, dans la présente description, une plage de valeurs préférées de pression peut être combinée avec une plage de valeurs de température plus préférées.

Dans la suite, des modes de réalisation particuliers de l'invention peuvent être décrits. Ils pourront être mis en œuvre séparément ou combinés entre eux, sans limitation de combinaisons lorsque c'est techniquement réalisable.

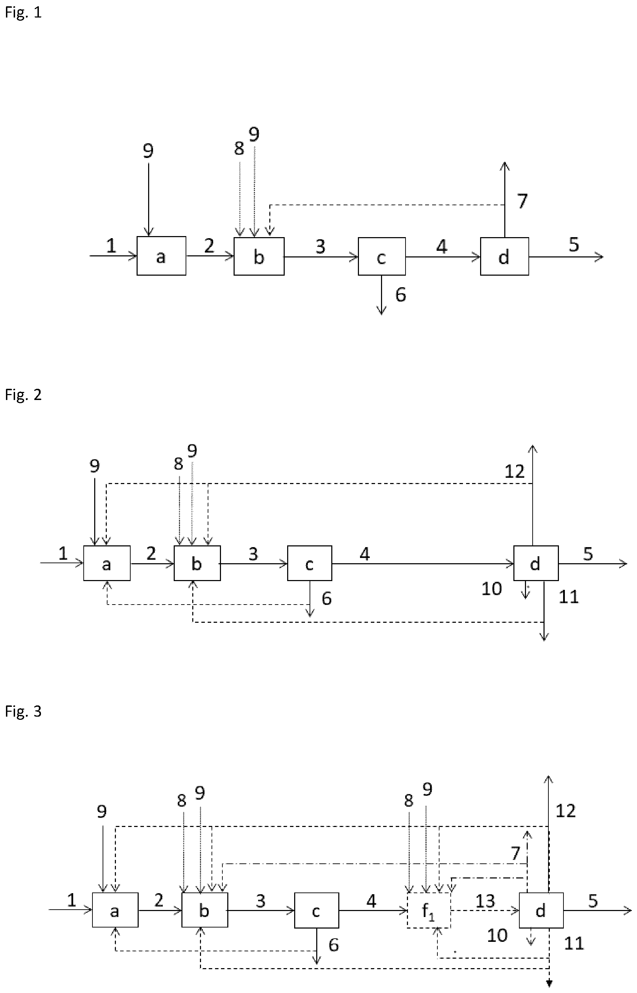

La description du procédé selon l'invention ci-dessous se réfère aux schémas des figures 1 à 13, illustrant différentes mises en œuvre du procédé selon l'invention.

Conformément à l'invention, le procédé de récupération d'acide phtalique et d'un plastique PVC cible réutilisable à partir d'une charge de PVC contenant au moins un phtalate, comporte, et peut consister en, les étapes suivantes :
a) une extraction solide-liquide d'une charge de PVC sous forme de particules 1 par mise en contact des particules de ladite charge avec un solvant 9 comportant au moins un alcool de formule CₙH₂ₙ₊₁OH, n entier positif inférieur à 4 ou supérieur à 8, pour produire une phase liquide enrichie en ledit phtalate et une phase solide comportant du plastique PVC appauvri en ledit phtalate ;
b) la transformation chimique dudit phtalate de la phase liquide en dialkylphtalate de formule C₆H₄(COOCₙH₂ₙ₊₁)₂ par transestérification au moyen dudit alcool (alcoolyse) pour enrichir ladite phase liquide en ledit dialkylphtalate ;
c) une séparation solide-liquide entre ladite phase solide et ladite phase liquide pour produire au moins un flux solide comportant le plastique PVC appauvri en ledit phtalate 6 afin de récupérer le plastique PVC cible ;
d) une séparation (gaz-liquide ou liquide-liquide) de la phase liquide, pour produire au moins un premier effluent liquide comportant le dialkylphtalate et un deuxième effluent liquide comprenant le solvant ;
g) la transformation chimique du dialkylphtalate obtenu lors de l'étape d) en acide phtalique de formule C₆H₄(COOH)₂ par hydrolyse en présence d'eau, pour produire au moins un effluent comportant une phase aqueuse enrichie en ledit acide phtalique ;
h) une étape de séparation dudit effluent obtenu à l'étape g) pour produire au moins un flux solide d'acide phtalique 20 afin de récupérer l'acide phtalique.

Les étapes a) à d), et optionnellement les étapes e) et f₁) et/ou f₂), permettent d'obtenir, notamment par la mise en œuvre d'une réaction d'alcoolyse, au moins un produit intermédiaire de type dialkylphtalate DAP (flux 5 ou 16 dans les figures) et au moins un flux solide comportant le plastique PVC appauvri en ledit phtalate 6 afin de récupérer le plastique PVC cible.

Lesdites étapes a), b), c), d), e), f₁) et f₂) sont également décrites dans la demande de brevet français déposée sous le numéro 21/05.299.

Les étapes g) et h) permettent d'obtenir, notamment par la mise en œuvre d'une réaction d'hydrolyse, au moins un flux solide comportant l'acide phtalique 20 afin de récupérer l'acide phtalique.

### Charge

Le procédé selon l'invention est alimenté par une charge appelée « charge de PVC » 1 comprenant au moins un plastique PVC, lequel comprend nécessairement au moins un phtalate tel que décrit dans la présente invention.

Ledit plastique PVC peut comporter au moins 0,1% massique de phtalates, voire au moins 1% massique de phtalates ou encore au moins 5% massique de phtalates. En général, les plastiques PVC comprennent avantageusement moins de 60% en masse de phtalates, typiquement moins de 40% en masse de phtalates.

Ladite charge de PVC est avantageusement une charge de PVC à recycler de type « chutes de production », i.e. des déchets issus des procédés de production du polymère PVC lors de sa polymérisation ou du plastique PVC lors de sa formulation/mise en forme ou de l'objet à base de PVC lors de sa production, ou de type « déchets post-consommation », i.e. des déchets générés après consommation par l'utilisateur dudit objet à base de PVC.

En particulier, la charge de PVC à recycler peut être issue de toute filière de collecte et de tri ou réseau existant pour les chutes de production et/ou les déchets post-consommation permettant d'isoler un flux à base d'au moins un plastique PVC comprenant au moins un phtalate, notamment les filières de collecte et de tri ou réseaux propres aux déchets plastiques.

Ainsi, la charge de PVC, qui est typiquement de type « chutes de production » et/ ou de type « déchets post-consommation », provient en général des grands domaines d'application utilisateurs du plastique PVC comme, et de façon non exhaustive, les domaines du bâtiment et de la construction, des emballages, de l'automobile, des équipements électriques et électroniques, des sports, des équipements médicaux, etc. De préférence, la charge de PVC provient du domaine du bâtiment et de la construction. Plus précisément, les objets à base de PVC sont en général utilisés dans ces domaines comme profilés (fenêtres, portes, stores, coffres de volets roulants), tuyaux et raccords, divers rigides et bouteilles, plaques et films rigides, films et feuilles souples, tubes et profilés souples, câbles, revêtements de sol, tissus enduits, etc. De préférence, les objets à base de PVC formant la charge de PVC comprennent au moins du PVC dit souple, c'est à dire du PVC contenant des additifs de type plastifiants, de préférence de type phtalate, comme c'est le cas par exemple pour les objets à base de PVC suivants : films et feuilles souples, tubes et profilés souples, câbles, revêtements de sol, tissus enduits, etc.

Avantageusement, la charge de PVC comprend au moins 50% massique, de préférence au moins 70% massique, de manière préférée au moins 90% massique et de manière encore plus préférée au moins 95% massique de plastique PVC comprenant au moins un phtalate.

De préférence, la charge de PVC comprend du PVC dit souple, c'est à dire du PVC contenant des additifs de type plastifiants, de préférence de type phtalate.

De façon encore plus préférée, la charge de PVC comprend majoritairement, voire exclusivement du PVC dit souple, c'est à dire du PVC contenant des additifs de type plastifiants, de préférence de type phtalate.

La charge de PVC traitée dans le procédé de récupération d'un DAP et d'un plastique PVC cible réutilisable selon l'invention est sous forme de particules. Ainsi, si la charge de PVC est sous une forme initiale qui est celle propre aux chutes de production ou aux déchets post-consommation, notamment dans ce dernier cas sous la forme initiale des objets à base de PVC, elle peut subir, au préalable, une étape de conditionnement comprenant au moins un broyage ou un déchiquetage pour former une charge de PVC sous forme de particules. En fonction des filières et/ou réseaux dont ces chutes de production et/ou objets à base de PVC en fin de vie sont issus, les déchets PVC peuvent être broyés et/ou lavés et/ou subir toute autre étape de conditionnement telle que décrite plus bas, afin de former la charge de PVC sous forme de particules adaptées au procédé selon l'invention. Par exemple, la charge de PVC peut avantageusement être sous la forme de broyats, éventuellement lavés, dont la plus grande dimension est inférieure à 20 cm, de préférence inférieure à 10 cm, de façon préférée inférieure à 1 cm et de façon encore plus préférée inférieure à 5 mm. La charge de PVC peut aussi être avantageusement sous forme de solide micronisé, c'est-à-dire sous forme de particules de préférence ayant une taille moyenne inférieure à 1 mm, par exemple comprise entre 10 micromètres (µm) et 800 micromètres (µm). La taille moyenne correspond avantageusement au diamètre moyen des sphères circonscrites auxdites particules.

Ainsi, par charge de PVC sous forme de particules, on entend des particules de plastique PVC ayant typiquement une taille moyenne, telle que définie précédemment, comprise entre 10 µm et 20 cm, par exemple des particules de type broyats ayant une taille moyenne comprise entre 1 mm et 20 cm, de préférence comprise entre 1 mm et 10 cm, plus préférentiellement comprise entre 1 mm et 1 cm, encore plus préférentiellement comprise entre 1 mm et 5 mm, ou des particules issues d'une micronisation (broyage très fin pour produire une poudre) d'une taille moyenne inférieure à 1 mm, de préférence comprise entre 10 µm et 800 µm.

De préférence, la charge de PVC traitée dans le procédé selon l'invention est sous forme de particules de type broyats, de préférence des particules de taille moyenne comprise entre 1 mm et 5 mm, ou des particules issues d'une micronisation (broyage très fin pour produire une poudre) d'une taille moyenne inférieure à 1 mm.

La charge de PVC peut également comprendre des impuretés « macroscopiques », comme du verre, du métal, des plastiques autres que le PVC (par exemple PET, etc.), du bois, du papier, du carton, des éléments minéraux, etc. Avantageusement, la charge de PVC comprend au plus 50% massique, de préférence au plus 30% massique, de manière préférée au plus 10% massique et de manière encore plus préférée au plus 5% massique d'impuretés « macroscopiques ».

Avantageusement, la charge de PVC sous forme de particules présente une teneur en eau inférieure ou égale à 0,3% massique, et de préférence inférieure ou égale à 0,1% massique.

Les différentes étapes du procédé selon l'invention menant à l'acide phtalique et au plastique PVC cible réutilisable sont détaillées dans les paragraphes qui suivent.

### Etape préalable optionnelle de conditionnement de la charge de PVC

Selon l'invention, le procédé peut comprendre une étape préalable de conditionnement de la charge de PVC (non représentée dans les figures) comportant au moins une étape de broyage ou de déchiquetage de la charge de PVC pour former une charge de PVC sous forme de particules solides telle que définie plus haut, apte à être envoyée à l'étape a) d'extraction solide-liquide. Cette étape préalable de conditionnement peut en outre comprendre une ou plusieurs étapes mentionnées dans la liste non exhaustive suivante : broyage par micronisation, tri, sur tri, lavage, séchage, etc. En fonction de la nature de la charge de PVC traitée, l'étape ou les étapes, ainsi que leurs fréquences et enchainements possibles, impliquées dans l'étape préalable de conditionnement sont notamment choisies par l'Homme du métier de façon à limiter la quantité d'impuretés macroscopiques et à réduire la taille des éléments solides composant initialement la charge de PVC.

Par exemple, l'étape préalable de conditionnement permet de fournir une charge de PVC sous forme de particules, par exemple de broyats, lavées, de taille moyenne inférieure à 5 mm, dont la teneur en impuretés macroscopiques est de préférence au plus de 10% massique, et de façon plus préférée au plus de 5% massique. Ladite charge de PVC préalablement conditionnée peut également être sous la forme de particules solides micronisées, c'est-à-dire sous la forme de particules ayant une taille moyenne inférieure à 1 mm, par exemple comprise entre 10 µm et 800 µm.

L'étape préalable de conditionnement de la charge de PVC comprend de préférence au moins une étape de séchage de la charge de PVC déjà sous forme de particules solides de taille et de teneur en impuretés macroscopiques *ad hoc,* telle que ladite charge de PVC contienne une teneur en eau résiduelle d'au plus 0,3 % massique et de préférence d'au plus 0,1% massique.

### Production du PVC cible et d'un flux isolé de DAP

### Étape a) d'extraction solide-liquide des phtalates

Le procédé selon l'invention comprend une étape a) d'extraction solide-liquide du ou des phtalates de la charge de PVC sous forme de particules 1 par la mise en contact de ladite charge avec un solvant 9 comportant un alcool de formule brute CₙH₂ₙ₊₁OH, n < 4 ou n > 8, afin d'obtenir un effluent 2 comprenant au moins une phase liquide et une phase solide. Ladite phase liquide est alors enrichie en ledit ou lesdits phtalates, et la phase solide comporte du plastique PVC appauvri en ledit ou lesdits phtalates.

Le choix spécifique de n pour l'alcool du solvant (exclusion des alcools en C4, C5, C6, C7, C8) permet, lors de l'étape b), de transformer, par transestérification au moyen dudit alcool, lesdits phtalates en au moins un DAP tel que défini plus loin, qui ne fait pas partie des phtalates non désirables tels que ceux soumis à autorisation par la règlementation REACH discutés plus haut.

Selon un ou plusieurs modes de réalisation, ledit alcool est un alcool de formule brute CₙH₂ₙ₊₁OH avec n < 4 , par exemple choisi dans la liste constituée par le méthanol, l'éthanol, le n-propanol, le *i-*propanol, et de façon encore plus préférée n = 1, ledit alcool étant alors du méthanol CH₃OH.

Selon un ou plusieurs modes de réalisation, ledit alcool est un alcool de formule brute CₙH₂ₙ₊₁OH avec n > 8, par exemple choisi dans la liste constituée par le nonanol, linéaire ou ramifié, le décanol, linéaire ou ramifié, le undécanol, linéaire ou ramifié, le dodécanol, linéaire ou ramifié , et de préférence le nonanol ou le décanol.

Selon un ou plusieurs modes de réalisation, ledit alcool est un alcool de formule brute CₙH₂ₙ₊₁OH avec n > 8 et n inférieur ou égal à 20, voire inférieur ou égal à 15.

Selon un ou plusieurs modes de réalisation, ledit alcool de formule brute CₙH₂ₙ₊₁OH, n < 4 ou n > 8 peut être utilisé selon l'invention sous sa forme alcoolate, c'est à dire sous la forme de la base conjuguée dudit alcool de formule brute CₙH₂ₙ₊₁O⁻, avec n < 4 ou n > 8, le contre-ion cationique, y compris de nature métallique, assurant l'électronégativité de ladite base conjuguée étant bien connu de l'Homme du métier.

Le solvant 9 peut en outre comprendre un co-solvant organique, additionné audit alcool, ce qui aide à l'extraction du ou des phtalates de la charge de PVC 1. Dans ce cas, ledit co-solvant organique peut être un ester dérivé dudit alcool, ledit ester ayant pour formule R'COOCₙH₂ₙ+1, n étant identique au n de l'alcool dont l'ester est issu (n < 4 ou n > 8, et par exemple n inférieur ou égal à 20), et R' étant un groupement alkyle (linéaire, ramifié ou cyclique, et de préférence linéaire), par exemple comprenant entre 1 et 3 atomes de carbone, par exemple 1 ou 2 atomes de carbone, ou bien ledit co-solvant organique peut être un éther tel que, et de façon non exhaustive, le cyclopentylméthyléther (CPME), le di-n-propyl ether, le dioxane, et de préférence le CPME.

Ledit co-solvant organique est additionné audit alcool de sorte que le ratio massique du co-solvant par rapport à l'alcool (co-solvant / solvant) soit compris entre 0 et 4, de préférence compris entre 0,01 et 4, plus préférentiellement compris entre 0,02 et 0,66, et de façon encore plus préférée entre 0,05 et 0,66.

Ledit co-solvant organique additionnel est avantageusement choisi, de préférence quand ledit alcool est le méthanol, dans le groupe constitué par l'acétate de méthyle, le propanoate de méthyle, le CPME.

De préférence, l'étape a) d'extraction solide-liquide du ou des phtalates de la charge de PVC 1 est réalisée par la mise en contact de ladite charge 1, sous forme de particules, avec du méthanol additionné de propanoate de méthyle, de préférence tel que le ratio massique entre le propanoate de méthyle et le méthanol soit compris entre 0 et 4, de préférence compris entre 0,01 et 4, plus préférentiellement compris entre 0,02 et 0,66, et de façon encore plus préférée entre 0,05 et 0,66. Dans ce cas, le DAP produit par le procédé est le diméthylphtalate (DMP).

L'étape a) d'extraction solide-liquide du ou des phtalates de la charge de PVC 1 est réalisée de préférence selon les conditions opératoires suivantes : une température comprise entre la température ambiante et 200°C, de préférence comprise entre 40°C et 180°C, de façon plus préférée comprise entre 60°C et 150°C, et de manière encore plus préférée entre 60°C et 145°C, une pression comprise entre la pression atmosphérique et 11,0 MPa, de préférence entre la pression atmosphérique et 5,0 MPa, de façon plus préférée entre la pression atmosphérique et 2,0 MPa, un temps de séjour compris entre 1 min et 10 h, de préférence entre 10 min et 4 h, de façon plus préférée entre 10 min et 2 h.

De préférence, l'étape a) est réalisée de sorte que le rapport molaire entre la quantité de l'alcool du solvant 9 et la quantité du ou des phtalates à extraire de la charge de PVC 1 soit compris entre 2 et 250, de préférence compris entre 4 et 90, et de façon encore plus préférée entre 4 et 30.

Le réacteur de l'étape a) du procédé selon l'invention peut avantageusement être un réacteur de type agité par un système d'agitation mécanique et/ou par boucle de recirculation et/ou par fluidisation, et/ou par ultrasons par exemple un réacteur de type discontinu (aussi appelé « batch ») ou continu, de préférence parfaitement agité, ou un réacteur de type tambour rotatif.

En matière de mise en œuvre, la charge de PVC sous forme de particules 1 et le solvant 9 comportant l'alcool, éventuellement additionné d'au moins un co-solvant organique, sont avantageusement mélangés.

Selon une première option, ledit mélange peut être réalisé préalablement à l'introduction de la charge de PVC et du solvant dans le réacteur de l'étape a) d'extraction solide-liquide. Dans ce cas, ledit mélange peut être formé dans un mélangeur et peut ensuite être introduit dans le réacteur, ce dernier étant maintenu à une pression et une température désirées.

Selon une deuxième option, la charge de PVC sous forme de particules 1 et le solvant 9 comportant l'alcool, éventuellement additionné d'au moins un co-solvant organique, peuvent être introduits séparément dans le réacteur de l'étape a) du procédé selon l'invention. Ladite charge de PVC solide et le solvant sont alors de préférence injectés dans le réacteur par deux lignes distinctes, l'une permettant d'injecter le solvant 9, et l'autre la charge de PVC solide sous forme de particules 1. Dans ce cas, le mélange de la charge de PVC et du solvant se forme directement dans ledit réacteur. Conformément à l'invention, ladite étape a) d'extraction solide-liquide permet l'obtention d'au moins un effluent 2 comprenant au moins une phase liquide contenant au moins les phtalates extraits et au moins une phase solide contenant le plastique PVC appauvri en phtalates, de préférence exempt de phtalates.

### Étape b) de transformation chimique desdits phtalates par réaction de transestérification (alcoolyse)

Le procédé selon l'invention comprend une étape b) de transformation chimique du ou des phtalates extraits à l'étape a) en au moins un DAP de formule C₆H₄(COOCₙH₂ₙ₊₁)₂ par réaction de transestérification (alcoolyse), de préférence en phase liquide, entre le ou lesdits phtalates de la phase liquide issue de l'étape a) et l'alcool de formule brute CₙH₂ₙ₊₁OH, avec n < 4 ou n > 8, de préférence avec n < 4, de façon encore plus préférée avec n = 1, ledit alcool étant alors du méthanol CH₃OH. Dans le cas où ledit alcool est du méthanol, ladite réaction de transestérification est alors dénommée réaction de méthanolyse.

L'étape b) de transformation chimique du ou des phtalates présents dans la phase liquide à l'issue de l'étape a) en un DAP de formule C₆H₄(COOCₙH₂ₙ₊₁)₂ par réaction de transestérification est de préférence réalisée selon les conditions opératoires suivantes : une température comprise entre la température ambiante et 200°C, de préférence comprise entre 40°C et 180°C, de façon plus préférée comprise entre 60°C et 150°C, une pression comprise entre la pression atmosphérique et 11,0 MPa, de préférence entre la pression atmosphérique et 5,0 MPa, de façon plus préférée entre la pression atmosphérique et 2,0 MPa, un temps de séjour compris entre 1 min et 10 h, de préférence entre 10 min et 4 h, de façon plus préférée entre 10 min et 2 h.

De préférence, l'étape b) est réalisée de sorte que le rapport molaire entre la quantité de l'alcool du solvant 9 et la quantité de phtalates à transformer de la phase liquide contenant le ou les phtalates extraits à l'issue de l'étape a) soit compris entre 2 et 250, de préférence compris entre 4 et 90 et de façon encore plus préférée entre 4 et 30.

L'alcool utilisé pour réaliser l'étape b) est le même que celui utilisé pour réaliser l'étape a).

De préférence, ladite étape b) de transformation chimique du ou des phtalates extraits à l'étape a) en un DAP de formule C₆H₄(COOCₙH₂ₙ₊₁)₂ par réaction de transestérification est réalisée en présence d'un catalyseur de transestérification, avantageusement introduit dans le milieu réactionnel.

Le catalyseur de transestérification 8 ainsi utilisé est par exemple choisi parmi les catalyseurs de la liste non exhaustive suivante, bien connue de l'Homme du métier, et de préférence dans la liste constituée par :
- les catalyseurs homogènes tels que les catalyseurs basiques (hydroxyde de sodium ou de potassium, méthylate de sodium ou de potassium, carbonate de sodium ou de potassium, etc.), les catalyseurs acides de Brônsted minéraux (acides chlorhydrique, sulfurique, phosphorique, etc.), les catalyseurs acides de Brônsted organiques (acides méthanesulfonique, trifluorométhanesulfonique, trifluoroacétique, etc.), les catalyseurs acides de Lewis dont notamment les composés du bore (BH₃, BF₃) et de l'aluminium (AlF₃, AlCl₃), et les composés organométalliques ;
- les catalyseurs hétérogènes tels que les oxydes de métaux alcalino-terreux (CaO, BaO, etc.), les carbonates ou hydrogéno-carbonates de métaux alcalins et/ou alcalino-terreux (CaCO₃, etc.), les métaux alcalins supportés sur alumines ou zéolites, les oxydes de zinc et leurs mélanges avec d'autres oxydes (par exemple oxyde de zinc et alumine), les résines échangeuses d'ions (cations ou anions), comme par exemple les résines sulfoniques, etc.

Par exemple, le catalyseur utilisé selon l'invention est un catalyseur homogène, notamment un catalyseur homogène de type catalyseur basique comme le méthylate de sodium.

De préférence, la quantité de catalyseur introduite est telle que le ratio massique entre le catalyseur et le ou les phtalates à transformer est compris entre 0,5% et 10% massique, de préférence entre 1% et 8% massique et de façon encore plus préférée entre 1% et 5% massique.

Le catalyseur, qu'il soit homogène ou hétérogène, peut être recyclé et/ou éliminé dans le procédé selon les méthodes bien connues de l'Homme du métier, et est de préférence recyclé. Il peut être isolé, pour être éliminé ou de préférence recyclé pour la réaction de transestérification, dans les étapes aval du procédé, ou lors de toute autre étape dédiée.

Le réacteur de l'étape b) du procédé selon l'invention peut avantageusement être un réacteur de type agité par un système d'agitation mécanique et/ou par boucle de recirculation et/ou par fluidisation, et/ou par ultrasons par exemple un réacteur de type discontinu ou continu, de préférence parfaitement agité, ou un réacteur de type tambour rotatif.

Conformément à l'invention, ladite étape b) de transformation des phtalates permet l'obtention d'au moins un effluent comprenant au moins une phase liquide contenant au moins le DAP de formule C₆H₄(COOCₙH₂ₙ₊₁)₂ obtenu après réaction de transestérification, c'est-à-dire la phase liquide formée à l'issue de l'étape a) et enrichie à l'étape b) en DAP.

Les étapes a) et b) du procédé selon l'invention peuvent être mises en œuvre au sein d'une même opération unitaire ou bien faire l'objet de deux opérations unitaires distinctes et consécutives, l'opération unitaire de l'étape a) étant alors toujours réalisée antérieurement à l'opération unitaire de l'étape b).

Dans les modes de réalisation représentés aux figures 1 à 5, les étapes a) et b), bien que figurées sous forme d'étapes séparées (« boîtes » séparées représentées), peuvent être mises en œuvre soit au sein d'une même opération unitaire, soit faire l'objet de deux opérations unitaires distinctes et consécutives. Dans le premier cas, l'effluent 2 est présent au sein du même réacteur utilisé par exemple pour réaliser les deux étapes a) et b).

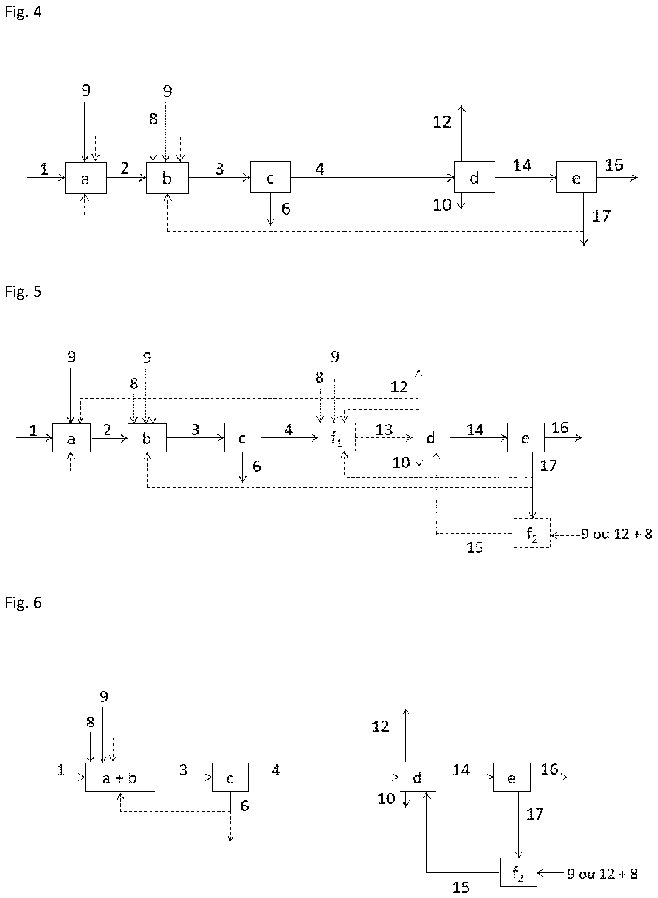

Dans le mode de réalisation représenté à la figure 6, qui est un des modes préférés selon l'invention, les étapes a) et b) font l'objet d'une même opération unitaire, ce qui est cette fois figuré par l'emploi d'une seule étape (a+b) (une seule « boîte » (a+b) représentée).

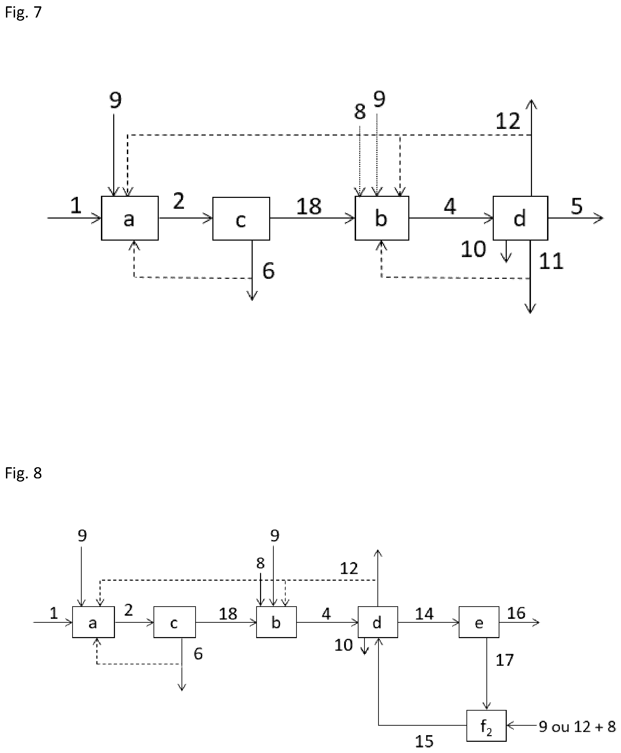

Dans les modes de réalisation représentés aux figures 7 et 8, celui de la figure 8 étant un des modes préférés selon l'invention, les étapes a) et b) font l'objet de deux opérations unitaires distinctes et consécutives, correspondant à un schéma dans lequel l'étape c) est réalisée entre les étapes a) et b), tel que décrit plus bas.

### Etape c) de séparation solide-liquide pour l'obtention d'un flux solide comportant le plastique PVC appauvri en phtalates

Le procédé selon l'invention comprend une étape c) de séparation solide-liquide entre d'une part la phase liquide contenant le ou les phtalates extraits à l'étape a) et/ou le DAP de formule C₆H₄(COOCₙH₂ₙ₊₁)₂ obtenu après réaction de transestérification à l'étape b), et d'autre part la phase solide contenant le plastique PVC appauvri en phtalates, de préférence exempt de phtalates.

La séparation physique de la phase liquide et de la phase solide peut avantageusement être mise en œuvre selon les techniques connues de l'Homme du métier telles que, et de façon non exhaustive, la filtration, la centrifugation, la précipitation électrostatique ou la décantation, lesdites techniques étant utilisées seules ou en combinaison, dans un ordre indifférent.

Cette étape c) de séparation solide-liquide permet donc de produire au moins un flux solide (6) comportant le plastique PVC appauvri en le ou les phtalates extraits à l'étape a), afin de récupérer ledit plastique PVC cible réutilisable.

L'obtention du PVC cible réutilisable tel que défini selon l'invention peut nécessiter de renvoyer tout ou partie du flux solide (6) obtenu à l'étape c) vers l'étape a), selon autant de cycles que nécessaires afin de produire ledit plastique PVC cible.

Cette possibilité de recyclage du flux solide est représentée aux figures 2 à 8.

Selon une première variante du procédé selon l'invention, ladite étape c) de séparation solide-liquide intervient après la réalisation des étapes a) et b). Cette première variante est illustrée aux figures 1 à6. Dans ce cas, l'effluent liquide 3 issu de l'étape b) est envoyé à l'étape c) de séparation solide-liquide qui conduit à la séparation entre la phase liquide contenant au moins le DAP obtenu après réaction de transestérification à l'étape b), et la phase solide contenant le plastique PVC appauvri en phtalate(s). Avantageusement pour cette première variante du procédé selon l'invention, les étapes a) et b) sont mises en œuvre conjointement au sein d'une même opération unitaire, cette mise en œuvre spécifique conduisant à une réduction du nombre d'opérations unitaires nécessaires à la réalisation du procédé selon l'invention et donc à une limitation du nombre d'équipements, de la quantité de solvant utilisé, de l'énergie engagée, etc., et donc une diminution des coûts. Un exemple préféré de mise en œuvre selon cette variante est illustré à la figure 6.

Selon une deuxième variante du procédé selon l'invention, l'étape c) de séparation solide-liquide intervient après la réalisation de l'étape a) et avant la réalisation de l'étape b). Cette deuxième variante est en particulier illustrée aux figures 7 et 8. Dans ce cas, l'effluent liquide 2 issu de l'étape a) est envoyé à l'étape c) de séparation solide-liquide qui conduit à la séparation de la phase liquide contenant les phtalates extraits de la phase solide contenant le PVC appauvri en phtalate(s). Conséquemment pour cette deuxième variante, les étapes a) et b) font l'objet de deux opérations unitaires distinctes. L'étape c) produit donc le flux solide 6 comportant le plastique PVC appauvri en phtalate(s), et un premier flux liquide 18 qui contient le ou les phtalates extraits à l'étape a) et qui est alors envoyé à l'étape b) pour la transformation du ou desdits phtalates par transestérification. Cette deuxième variante est particulièrement adaptée dans le cas où la charge de PVC à traiter conduirait à la formation, lors de l'étape a), d'une phase solide non favorable à la réalisation de la réaction chimique de transestérification (en matière de propriétés chimiques ou rhéologique, etc.).

Pour exemple, selon des modes de réalisation conformes à cette deuxième variante du procédé comme représentés aux figures 7 et 8, dans lesquels l'étape c) est réalisée entre les étapes a) et b), les étapes a) et c) selon l'invention peuvent être consécutivement mises en œuvre dans un même réacteur discontinu possédant un dispositif de filtration de l'effluent liquide 2 permettant plusieurs cycles d'extraction des phtalates de la phase solide et d'un dispositif de soutirage d'au moins la phase solide 6 permettant la récupération finale du plastique PVC cible.

Pour autre exemple, l'étape c) peut se faire par centrifugation de l'effluent liquide 2 ou 3 comprenant la phase liquide contenant au moins les phtalates extraits et/ou le DAP et de la phase solide issue de l'étape a), conduisant à la séparation dudit solide 6, et avantageusement au renvoi de tout ou partie dudit solide à l'étape a), de préférence préalablement mis en suspension, par exemple au moyen d'un appoint de solvant 9 (non représenté dans les figures), jusqu'à produire le plastique PVC cible réutilisable.

### Etape d) de séparation pour l'obtention d'un premier effluent liquide comportant le dialkylphtalate

Le procédé selon l'invention comprend une étape d) de séparation gaz-liquide ou liquide-liquide permettant d'extraire le DAP de formule C₆H₄(COOCₙH₂ₙ₊₁)₂ de la phase liquide obtenue à l'issue de la mise en œuvre d'au moins les étapes a), b) et c).

Un flux liquide (4, 13) contenant ladite phase liquide alimente avantageusement cette étape d) de séparation qui permet ainsi de produire au moins un premier effluent liquide comportant le DAP (flux 5 ou 14 selon les figures) et un deuxième effluent comprenant au moins ledit solvant (flux 7 ou 12 selon les figures). Ledit deuxième effluent est sous forme liquide, y compris si la séparation est une séparation gaz-liquide, la phase gaz pouvant être condensée pour former le deuxième effluent liquide.

L'étape d) de séparation peut être réalisée selon les méthodes bien connues de l'Homme du métier telles que, et de façon non exhaustive, la distillation, la décantation, l'évaporation, l'extraction liquide-liquide, etc., réalisées seules ou en combinaison. Les conditions opératoires de cette étape (température, pression, etc.) sont déterminées en fonction de la méthode de séparation choisie. Selon un ou plusieurs modes de réalisation, le premier effluent 5 consiste essentiellement en ledit DAP. Dans ce(s) cas, le deuxième effluent liquide 7, représenté par exemple à la figure 1 (ou en tant qu'option à la figure 3), consiste en la phase liquide résiduelle après extraction du DAP, qui contient au moins le solvant, c'est-à-dire l'alcool éventuellement additionné de co-solvant, les sous-produits de type alcool (AL_{A}), les alkylphtalates intermédiaires (API_{A}) et le ou les phtalates extraits à l'issue de l'étape a) du procédé selon l'invention éventuellement non convertis. Le deuxième effluent liquide 7 peut être renvoyé, en tout ou partie, de préférence totalement, à l'étape b) du procédé selon l'invention.

Il est également possible dans ce(s) cas, notamment en fonction des méthodes de séparation choisies, par exemple une distillation avec un soutirage latéral, un enchaînement de colonnes de distillation ou une extraction liquide-liquide, de séparer de la phase liquide non seulement le solvant, mais également les AL_{A} et très avantageusement les API_{A} avec éventuellement les phtalates extraits à l'étape a) et non convertis. Une telle séparation est par exemple illustrée à la figure 2 ou à la figure 7 (et comme alternative à la production d'un flux 7 à la figure 3), où l'on peut voir que l'étape d) produit, en plus du premier effluent 5 consistant essentiellement en ledit DAP et du deuxième effluent 12 consistant essentiellement en ledit solvant, un troisième effluent 10 comportant des AL_{A} obtenus lors de la transestérification à l'étape b), et un quatrième effluent 11 comportant du ou des phtalates convertis partiellement (API_{A}) et/ou non convertis à l'étape b) et éventuellement d'autres impuretés solubles. Le quatrième effluent 11 peut alors être avantageusement renvoyé à l'étape b) du procédé selon l'invention, notamment selon les première et deuxième variantes du procédé selon l'invention, de façon à poursuivre les réactions chimiques conduisant au DAP et à améliorer ainsi le rendement en ce produit.

Selon un ou plusieurs modes de réalisation alternatifs, tels que représentés aux figures 4 à 6 et à la figure 8, le premier effluent liquide 14 comportant le DAP comprend aussi d'autres composés comme du ou des phtalates convertis partiellement (API_{A}) et/ou non convertis à l'étape b) et éventuellement des impuretés solubles. Comme décrit plus bas, selon ce ou ces modes de réalisation, une étape de purification du DAP du premier effluent est nécessaire. Selon ce ou ces modes de réalisation, l'étape d) de séparation produit donc avantageusement ledit premier effluent liquide 14 de DAP non pur, un deuxième effluent 12 de préférence consistant essentiellement en ledit solvant, et de préférence un troisième effluent 10 comportant des AL_{A} obtenus lors de la transestérification à l'étape b). L'isolement des AL_{A} et du solvant est notamment rendu possible en fonction des méthodes de séparation choisies, par exemple une distillation avec un soutirage latéral, un enchaînement de colonnes de distillation ou une extraction liquide-liquide. Dans le cas où le deuxième effluent 12 consiste essentiellement en ledit solvant ainsi récupéré, le deuxième effluent 12 peut alors être avantageusement renvoyé, en partie ou en totalité, de préférence en totalité, à l'étape a) et/ou à l'étape b) du procédé selon l'invention, et notamment selon les première et deuxième variantes de procédé selon l'invention.

### Etape e) de purification du DAP (optionnelle)

Le procédé selon l'invention peut comprendre une étape e) optionnelle de purification du premier effluent 14 comprenant le DAP issu de l'étape d) de séparation, pour améliorer sa qualité et donc, au final, sa valorisation. Les modes de réalisation représentés aux figures 4, 5, 6 et 8 illustrent la mise en œuvre d'une telle étape e) de purification.

Dans le cas de la mise en œuvre de ladite étape e), le solvant a été avantageusement isolé lors de la mise en œuvre de l'étape d). Par ailleurs, les API_{A} et éventuellement le ou les phtalates extraits à l'issue de l'étape a) du procédé selon l'invention non convertis à l'issue de l'étape b) peuvent avoir été isolés lors de l'étape d) du procédé selon l'invention, ou encore être isolés lors de la mise en œuvre de ladite étape e) de purification.

Ainsi, il est possible d'envoyer le premier effluent 14, comprenant le DAP, du ou des phtalates partiellement convertis et/ou non convertis à l'étape b) et éventuellement des impuretés solubles, à cette étape de purification e) pour former un produit liquide 16 consistant essentiellement en ledit DAP, et un résidu liquide 17 comprenant le ou les phtalates partiellement convertis et/ou non convertis à l'étape b) et éventuellement les impuretés solubles.

Le résidu liquide 17 ainsi récupéré peut alors être avantageusement renvoyé à l'étape b) du procédé selon l'invention, notamment selon les première et deuxième variantes du procédé selon l'invention, de façon à poursuivre les réactions chimiques conduisant au DAP, comme illustré à la figure 4 ou à la figure 5.

L'étape e) de purification peut avantageusement être réalisée par des méthodes bien connues de l'Homme du métier, telles qu'une précipitation, une cristallisation, une adsorption, éventuellement suivies d'une filtration ou d'une centrifugation. L'étape e) de purification peut comprendre la mise en œuvre de plusieurs de ces méthodes en parallèle ou en série. Par exemple, et sans être exhaustif, l'étape de purification e) peut comprendre une étape de précipitation et filtration, suivie d'une étape d'adsorption, ou encore comprendre une étape d'adsorption et filtration, éventuellement suivie d'une étape de précipitation, ou encore comprendre une étape de cristallisation et filtration. Les conditions opératoires à cette étape e) (température, pression, etc.) sont déterminées en fonction de la méthode de purification choisie.

### Etape(s) supplémentaire(s) f₁) et/ou f₂) de transformation chimique des phtalates par transestérification (optionnelles)

Afin de favoriser la production du DAP selon l'invention, il est possible de réaliser, indépendamment de l'étape b) de transformation chimique du ou des phtalates extraits à l'étape a), une étape de transformation chimique additionnelle permettant la transformation des API_{A} et/ou du ou des phtalates extraits éventuellement non convertis à l'issue de l'étape b).

Le procédé peut ainsi comprendre en outre une étape supplémentaire f₁), tel que représenté à la figure 3 ou la figure 5, de transformation chimique par transestérification du ou des phtalates non convertis à l'étape b) et/ou d'au moins un API_{A} produit à l'étape b), en DAP de formule C₆H₄(COOCₙH₂ₙ₊₁)₂ au moyen du solvant comportant l'alcool. Dans ces modes de réalisation, l'étape f₁) est réalisée entre les étapes c) et d), et avantageusement après l'étape b), par envoi de la phase liquide 4, avantageusement obtenue à l'issue de l'ensemble des étapes a), b) et c), dans un premier réacteur de transestérification supplémentaire, pour produire un deuxième flux liquide 13 enrichi en DAP, ledit deuxième flux liquide 13 étant envoyé à l'étape d). Selon ce mode de réalisation, l'étape c) est de préférence mise en œuvre à l'issue de l'étape b).

Le procédé peut aussi comprendre une étape supplémentaire f₂) de transformation chimique par transestérification du ou des phtalates non convertis à l'étape b) et/ou d'au moins un API_{A} produit à l'étape b), ou éventuellement à l'étape f₁) optionnelle, en DAP de formule C₆H₄(COOCₙH₂ₙ₊₁)₂ au moyen du solvant comportant l'alcool, l'étape f₂) étant réalisée successivement à l'étape e) par envoi du résidu liquide 17 issu de l'étape e) dans un deuxième réacteur de transestérification supplémentaire pour produire un troisième flux liquide 15 enrichi en ledit DAP, ledit troisième flux liquide 15 étant renvoyé à l'étape d).

La mise en œuvre de l'étape supplémentaire f₁) et/ou de l'étape supplémentaire f₂) de transformation chimique par transestérification peut être réalisée selon la première variante (étape c) de séparation solide-liquide réalisée après les étapes a) et b)) ou deuxième variante (étape c) de séparation solide-liquide située entre les étapes a) et b)) du procédé selon l'invention.

De préférence, le procédé selon l'invention comprend une seule étape supplémentaire de transformation chimique par transestérification, et de préférence l'étape f₂).

La mise en œuvre de l'étape f₁) et/ou de l'étape f₂) est telle que décrite pour l'étape b) du procédé selon l'invention. En particulier, les gammes associées aux conditions opératoires des étapes b) et f₁) et/ou f₂) sont similaires, et ces dernières sont choisies par l'Homme du métier de façon à favoriser la production du DAP en fonction de la nature chimique du flux à traiter en entrée de ladite étape f₁) et/ou étape f₂).

Il en est de même pour l'utilisation préférée d'un catalyseur de transestérification 8, tel que décrit à l'étape b). Le catalyseur de transestérification à(aux) l'étape(s) f₁) et/ou f₂) peut être identique ou différent de celui utilisé à l'étape b).

Ledit flux envoyé à l'étape f₁) et/ou à l'étape f₂) (flux 4 ou résidu liquide 17) est une phase liquide comprenant un ou des phtalates extraits à l'étape a) et éventuellement partiellement convertis (API_{A}) et/ou non convertis à l'étape b), et éventuellement des impuretés solubles, qui sont ensuite isolés soit lors de la mise en œuvre de l'étape d) de séparation, soit lors de la mise en œuvre de l'étape e) de purification du procédé selon l'invention si cette dernière est avantageusement mise en œuvre.

En fonction des enchainements d'étapes considérés faisant intervenir l'étape f₁) et/ou l'étape f₂), il peut être nécessaire d'utiliser un apport complémentaire de solvant comprenant l'alcool de formule brute CₙH₂ₙ₊₁OH avec n < 4 ou n > 8, éventuellement additionné d'au moins un co-solvant organique, cet apport complémentaire en solvant pouvant résulter d'un appoint en solvant « frais » 9 ou bien d'un recyclage du flux 12 dudit solvant éventuellement isolé à l'issue de l'étape d) du procédé selon l'invention. Cet apport complémentaire dans le premier réacteur supplémentaire de transestérification mis en œuvre à l'étape f₁) et/ou dans le deuxième réacteur supplémentaire de transestérification mis en œuvre à l'étape f₂), par appoint de solvant frais 9, et/ou par recyclage du deuxième effluent 12 consistant en ledit solvant, est illustré aux figures 3, 5, 6 et 8.

Lorsque l'étape de purification e) est mise en œuvre, au moins une partie dudit résidu liquide 17 produit à l'étape e) peut être recyclée à l'étape f₁), comme illustré à la figure 5, de façon à poursuivre les réactions chimiques conduisant au DAP.

Les figures 6 et 8 représentent des modes de réalisation préférés selon respectivement la première variante (étape c) de séparation solide-liquide après la réalisation des étapes a) et b)) et selon la deuxième variante (étape c) de séparation solide-liquide entre les étapes a) et b)) du procédé selon l'invention.

Comme visible à la figure 6, selon un mode de réalisation préféré de l'invention conforme à la première variante, le procédé comporte une mise en œuvre au sein d'une même opération unitaire des étapes a) et b), une étape c) de séparation solide-liquide située après les étapes a) et b), une étape d) de séparation, une étape de purification e) d'un premier effluent 14 obtenu à l'étape d) comprenant le DAP, et avantageusement une étape supplémentaire de transestérification f₂) du résidu 17 issu de l'étape e).

Selon ce mode de réalisation, tel que schématisé en figure 6, la charge de PVC sous forme de particules 1, éventuellement préalablement conditionnée, est introduite dans un réacteur combinant la réalisation des étapes a) et b) respectivement d'extraction solide-liquide et de transformation chimique par transestérification de préférence en présence d'un catalyseur 8. Le réacteur est également alimenté par un flux de solvant 9 frais externe au procédé comprenant au moins un alcool de formule brute CₙH₂ₙ₊₁OH, avec n < 4 ou n > 8, de préférence du méthanol, additionné d'un éventuel co-solvant, de préférence du propanoate de méthyle, et optionnellement par au moins une fraction d'un flux 12 de solvant isolé à l'étape d) de séparation. L'effluent réactionnel 3 contenant la phase liquide comportant au moins le DAP, de préférence le DMP, et la phase solide comportant le plastique PVC appauvri en phtalates, de préférence exempt de phtalates, est envoyé à l'étape c) de séparation solide-liquide, par exemple mettant en œuvre une centrifugation, pour produire un flux solide 6 comportant ledit plastique PVC appauvri en le ou les phtalates extraits afin de récupérer ledit plastique PVC cible réutilisable, et un flux liquide 4 contenant au moins le DAP, de préférence le DMP, et au moins le solvant. Le flux solide 6 peut être recyclé en partie à l'étape a). Le flux liquide 4 issu de l'étape c), contenant le DAP, le solvant, éventuellement du ou des phtalates non convertis ou partiellement convertis (API_{A}) et éventuellement des AL_{A}, est envoyé à l'étape de séparation d) qui permet d'isoler d'une part le solvant selon un flux 12, mais aussi de préférence les AL_{A} selon un flux 10, et enfin un effluent liquide 14 comportant le DAP, de préférence du DMP, et éventuellement du ou des phtalates partiellement et/ou non convertis et éventuellement des impuretés solubles. L'effluent liquide 14 est envoyé vers une étape e) de purification, afin d'obtenir le DAP, de préférence le DMP, purifié. Le résidu 17 issu de cette étape de purification e) pouvant contenir encore du ou des phtalates non convertis ou partiellement convertis (API_{A}), une étape supplémentaire f₂) de transformation chimique de transestérification est de préférence réalisée. Le résidu 17 est donc avantageusement envoyé dans un deuxième réacteur de transestérification contenant un catalyseur de transestérification adapté, pour réaliser la transestérification du ou des phtalates non convertis ou partiellement convertis (API_{A}) au moyen d'un solvant 9 comprenant l'alcool de formule brute CₙH₂ₙ₊₁OH, avec n < 4 ou n > 8, additionné d'un éventuel co-solvant, de préférence du propanoate de méthyle. Le solvant peut être un appoint de solvant frais ou provenir du flux 12 recyclé au moins en partie à cette étape f₂). Cette étape f₂) produit un flux liquide 15 enrichi en ledit DAP, de préférence en DMP, renvoyé à l'étape d) de séparation.

Comme représenté à la figure 8, selon un autre mode de réalisation préféré de l'invention, conforme à la deuxième variante, le procédé comporte une mise en œuvre des étapes a) et b) selon deux opérations unitaires distinctes, avec une étape c) réalisée entre les étapes a) et b), suivies d'une étape d), et comporte également une étape de purification e) d'un premier effluent 14 obtenu à l'étape d) comprenant le DAP, et une étape supplémentaire de transestérification f₂) du résidu 17 issu de l'étape e).

Selon ce mode de réalisation, tel que schématisé en figure 8, la charge de PVC sous forme de particules 1, éventuellement préalablement conditionnée, est introduite dans un réacteur pour opérer l'étape a) d'extraction solide-liquide du ou des phtalates de ladite charge de PVC. Le réacteur est alimenté par un flux de solvant 9 frais externe au procédé comprenant au moins un alcool de formule brute CₙH₂ₙ₊₁OH, n entier avec n < 4 ou n > 8, de préférence du méthanol, additionné d'un éventuel co-solvant, de préférence du propanoate de méthyle, et optionnellement par un flux 12 de solvant isolé à l'étape d) ultérieure de séparation. L'effluent 2 produit à l'étape a) comprend au moins une phase liquide contenant au moins le ou les phtalates extraits de ladite charge 1 et au moins une phase solide contenant le plastique PVC appauvri en phtalates, de préférence exempt des phtalates extraits. L'effluent 2 est envoyé à une étape c) de séparation solide-liquide, par exemple mettant en œuvre une centrifugation, pour produire un flux solide 6 comportant ledit plastique PVC appauvri en le ou les phtalates, afin de récupérer ledit plastique PVC cible réutilisable, et un flux liquide 18 contenant au moins le ou les phtalates extraits à l'étape a) et au moins le solvant. Le flux liquide 18 est alors envoyé dans un réacteur pour effectuer l'étape b) de transformation chimique du ou des phtalates extraits par transestérification de préférence en présence d'un catalyseur 8. Le réacteur de transestérification peut être également alimenté par un flux de solvant 9 frais externe au procédé comprenant le même alcool, de préférence du méthanol, additionné d'un éventuel co-solvant, de préférence du propanoate de méthyle, et optionnellement par au moins une fraction d'un flux 12 de solvant isolé à l'étape d) de séparation. L'effluent réactionnel 4 contenant la phase liquide comportant au moins le DAP, de préférence le DMP, le solvant, du ou des phtalates non convertis ou partiellement convertis (API_{A}), est envoyé à l'étape de séparation d) qui permet d'isoler d'une part le solvant selon un flux 12, mais aussi les AL_{A} selon un flux 10, et enfin un effluent liquide 14 comportant le DAP, de préférence du DMP, et éventuellement du ou des phtalates partiellement (API_{A}) et/ou non convertis et éventuellement des impuretés solubles. L'effluent liquide 14 est envoyé de préférence vers une étape e) de purification, afin d'obtenir le DAP, de préférence le DMP, purifié 16. Le résidu 17 issu de cette étape de purification e) pouvant contenir encore du ou des phtalates non convertis ou partiellement convertis (API_{A}), une étape supplémentaire f₂) de transformation chimique transestérification est de préférence réalisée. Le résidu 17 est avantageusement envoyé dans un deuxième réacteur de transestérification contenant de préférence un catalyseur de transestérification adapté, pour réaliser la transestérification du ou des phtalates non convertis ou partiellement convertis (API_{A}) au moyen d'un solvant 9 comprenant l'alcool de formule brute CₙH₂ₙ₊₁OH, avec n < 4 ou n > 8, additionné d'un éventuel co-solvant, de préférence du propanoate de méthyle. Le solvant peut être un appoint de solvant frais ou provenir du flux 12 recyclé au moins en partie à cette étape f₂). Cette étape f₂) produit un flux liquide 15 enrichi en ledit DAP, de préférence en DMP, renvoyé à l'étape d) de séparation.

### Production d'acide phtalique solide

Les étapes g) et h) peuvent être mises en œuvre indifféremment à l'issue des divers modes de réalisation et variantes du procédé de l'invention pour la mise en œuvre des étapes a) à d), et éventuellement des étapes optionnelles associées, décrites ci-dessus.

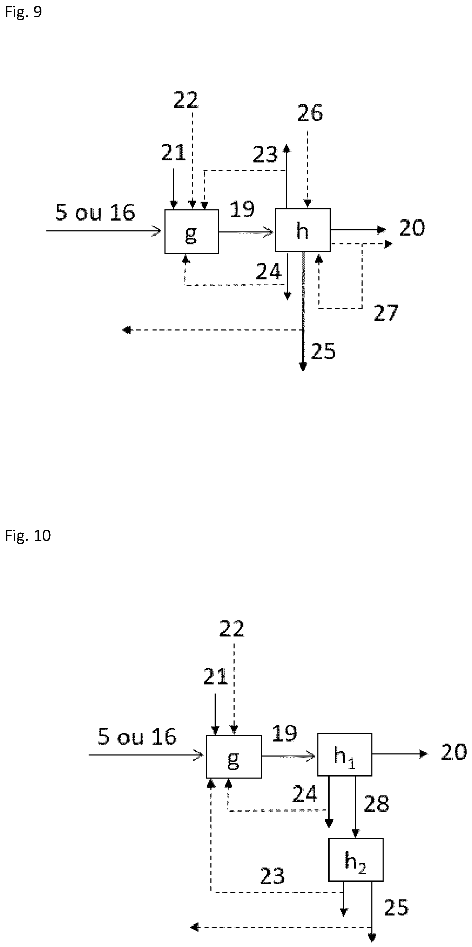

Pour exemple, il est ainsi possible de combiner le mode de réalisation illustré à la figure 3, exemple de réalisation des étapes a) à d) et de l'étape optionnelle f1) conduisant au flux 5 consistant essentiellement en le DAP, avec la figure 9 décrivant la mise en œuvre générale des dites étapes g) et h) pour l'obtention d'au moins un flux solide comportant l'acide phtalique.

Selon un aspect primordial de l'invention, l'acide phtalique est produit à partir du DAP produit à l'issue des étapes a) à d), et optionnellement e) et f1 et/ou f2), qui a été isolé, de manière à faciliter la production d'acide phtalique présentant une bonne pureté, selon les étapes g) et h) décrites en détail ci-dessous.

### Étape g) de transformation chimique du DAP obtenu à l'issue des étapes a) à d) par hydrolyse

Le procédé selon l'invention comprend une étape g) de transformation chimique du dialkylphtalate obtenu lors de l'étape d) (flux 5) ou lors de l'étape optionnelle e) (flux 16) en au moins l'acide phtalique de formule C₆H₄(COOH)₂ par réaction d'hydrolyse, de préférence en phase liquide, entre ledit DAP et de l'eau (H₂O).

L'étape g) d'hydrolyse du DAP est de préférence réalisée selon les conditions opératoires suivantes : une température comprise entre la température ambiante et 150°C, de préférence comprise entre la température ambiante et 145°C, plus préférentiellement comprise entre 40°C et 130°C, et de façon plus préférée comprise entre 60°C et 110°C, une pression comprise entre la pression atmosphérique et 5,0 MPa, de préférence entre la pression atmosphérique et 2,0 MPa, de façon plus préférée entre la pression atmosphérique et 0,5 MPa, un temps de séjour compris entre 1 min et 10 h, de préférence entre 10 min et 4 h, de façon plus préférée entre 10 min et 2 h et de façon encore plus préférée entre 10 min et 1 h.

De préférence, l'étape g) est réalisée de sorte que le rapport molaire entre la quantité d'eau 21 et la quantité de DAP à transformer soit compris entre 100 et 9000, de préférence compris entre 150 et 1800 et de façon encore plus préférée entre 200 et 850.

De préférence, ladite étape g) d'hydrolyse du DAP est réalisée en présence d'un catalyseur d'hydrolyse 22, avantageusement introduit dans le milieu réactionnel.

Le catalyseur d'hydrolyse 22 ainsi utilisé est avantageusement un catalyseur acide, par exemple choisi parmi les catalyseurs acides de la liste non exhaustive suivante, bien connue de l'Homme du métier, et de préférence dans la liste constituée par :
- les catalyseurs homogènes tels que les catalyseurs acides de Brönsted minéraux (e.g. acides chlorhydrique, sulfurique, phosphorique, etc.), les catalyseurs acides de Brônsted organiques (acides méthanesulfonique, trifluorométhanesulfonique, trifluoroacétique, para-toluènesulfonique, etc.), et les catalyseurs acides de Lewis notamment (e.g. AlF₃) ;
- les catalyseurs hétérogènes acides tels que les alumines, les alumines chlorées ou fluorées, les aluminosilicates mésoporeux, les zéolithes et leurs mélanges avec d'autres oxydes, les résines échangeuses d'ions (H⁺), comme par exemple les résines sulfoniques, etc.

Par exemple, le catalyseur utilisé selon l'invention est un catalyseur homogène, notamment un catalyseur homogène de type catalyseur acide de Brônsted organique comme l'acide p-toluènesulfonique.

De préférence, la quantité de catalyseur introduite est telle que le ratio massique entre le catalyseur et le DAP est compris entre 0,02% et 10% massique, de préférence entre 0,5% et 8% massique et de façon encore plus préférée entre 1% et 5% massique.

Le catalyseur, qu'il soit homogène ou hétérogène, peut être recyclé et/ou éliminé dans le procédé selon les méthodes bien connues de l'Homme du métier, et est de préférence recyclé. Il peut être isolé, pour être éliminé ou de préférence recyclé pour la réaction d'hydrolyse, dans les étapes aval du procédé ou lors de toute autre étape dédiée.

Le réacteur mis en œuvre à l'étape g) peut avantageusement être un réacteur de type agité par un système d'agitation mécanique et/ou par boucle de recirculation et/ou par fluidisation, et/ou par ultrasons, par exemple un réacteur de type discontinu ou continu, de préférence parfaitement agité, ou un réacteur de type tambour rotatif.

Conformément à l'invention, ladite étape g) d'hydrolyse du DAP permet l'obtention d'au moins un effluent 19 comprenant au moins une phase aqueuse contenant au moins l'acide phtalique de formule C₆H₄(COOH)₂ obtenu après réaction d'hydrolyse du DAP contenu initialement dans les flux 5 et 16 formés respectivement à l'issue des étapes d) et e) et envoyés à l'étape g).

### Étape h) de séparation d'au moins un effluent solide comportant l'acide phtalique

Le procédé selon l'invention comprend une étape h) comportant au moins une séparation solide-liquide opérée sur au moins l'effluent 19 issu de l'étape g), pour produire au moins un flux solide comprenant l'acide phtalique 20 afin de récupérer l'AP et au moins trois autres flux liquides 23, 24 et 25 comprenant respectivement et distinctement au moins l'eau résiduelle de l'étape d'hydrolyse g), au moins du DAP et/ou du API_{H} résultant de l'hydrolyse partielle dudit DAP, et au moins de l'AL_{H}, produit secondaire résultant de l'hydrolyse partielle ou totale du DAP.

L'acide phtalique sous forme solide, séparé à cette étape h) pour produire le flux solide d'acide phtalique 20, peut être formé à l'étape h) selon différentes mises en œuvre détaillées plus bas, en relation avec les figures 10 à 13.

La présente invention n'exclut pas qu'une partie d'acide phtalique sous forme solide soit produit à l'étape g).

Pour faciliter la réalisation de l'étape h), un solvant organique 26 non miscible à l'eau et présentant des affinités physico-chimiques avec au moins le DAP et/ou l'API_{H}, encore appelé « solvant de séparation », peut être employé pour faciliter l'obtention du flux solide comprenant l'acide phtalique 20. Ledit solvant de séparation non miscible à l'eau est avantageusement choisi dans la liste constituée par : les cétones, telle que la 2,4-dimethyl-3-pentanone, les éthers, tel que le méthoxycyclopentane (CPME), les hydrocarbures, notamment cycliques et aromatiques, tels que le toluène, les xylènes, l'isohexane, pris seul ou en mélange. De préférence, le solvant de séparation est choisi parmi la 2,4-diméthyl-3-pentanone, le CPME, le toluène et les xylènes. Lorsqu'un tel solvant de séparation est employé, ledit solvant est avantageusement séparé selon des méthodes bien connues de l'Homme du métier, et de préférence renvoyé à l'étape h) (flux 27).

De préférence, l'étape h) est opérée sans ajout de solvant de séparation additionnel.

Les étapes g) et h) du procédé selon l'invention sont représentées sur la figure générique 9.

La séparation physique solide-liquide de l'effluent 19 issu de l'étape g) peut avantageusement être mise en œuvre selon les techniques connues de l'Homme du métier telles que, et de façon non exhaustive, la filtration, la centrifugation, l'utilisation d'un agent précipitant, la précipitation électrostatique, ou la décantation, lesdites techniques étant utilisées seules ou en combinaison, dans un ordre indifférent. En particulier, selon le solvant employé lors des étapes a) à d) du procédé selon l'invention, et donc selon la nature chimique du DAP ainsi formé (formule brute C₆H₄(COOCₙH₂ₙ₊₁)₂ avec n < 4 ou n > 8), plusieurs étapes de séparation peuvent être judicieusement choisies et agencées pour produire au mieux les flux 20, 23, 24 et 25 susmentionnés.

Différents modes de réalisation de l'étape h) sont décrits ci-dessous en relation avec les figures 10 à 13.

Un premier exemple de mise en œuvre de l'étape h) est représenté à la figure 10, et est particulièrement bien adapté lorsque que le DAP envoyé à l'étape g) a pour formule chimique C₆H₄(COOCₙH₂ₙ₊₁)₂ avec n < 4, c'est-à-dire n = 1, 2 ou 3.

Selon cette configuration, l'effluent 19 peut comporter une seule phase liquide ou deux phases liquides immiscibles, en particulier l'effluent 19 comporte 1 seule phase liquide (liquide monophasique) pour n inférieur ou égal à 2.

L'effluent 19 est envoyé à une première étape **h₁)** combinant un changement de phase liquide-solide de l'acide phtalique et une première séparation solide-liquide du milieu ainsi obtenu pour l'obtention d'au moins un flux solide comprenant l'acide phtalique 20 (acide phtalique à l'état solide).

Ce changement de phase liquide-solide consiste en un changement de phase de l'acide phtalique de l'état dissous dans la phase aqueuse de l'effluent 19, obtenu à l'issu de l'étape g), à un état solide permettant sa récupération ultérieure lors de la première séparation solide-liquide de l'étape h₁). Le changement de de phase liquide-solide de l'acide phtalique peut avantageusement être mis en œuvre au moyen d'une ou plusieurs opérations de cristallisation ou précipitation selon les techniques connues de l'Homme du métier telles que, et de façon non exhaustive, une cristallisation à paroi froide, l'utilisation d'un agent précipitant, une distillation batch, etc., lesdites techniques étant utilisées seules ou en combinaison, dans un ordre indifférent. Par exemple le changement de phase de l'acide phtalique de l'état dissous à l'état solide est obtenu par un refroidissement à une température comprise entre 10°C et la température ambiante, par exemple à une température de 15°C, par exemple via l'emploi d'une cristallisation à paroi froide, de façon à provoquer ladite précipitation de l'acide phtalique, et produire une phase solide d'acide phtalique. Consécutivement à la formation de l'acide phtalique à l'état solide, une séparation gaz-liquide ou liquide-liquide, selon des techniques bien connues de l'homme du métier comme la distillation, la décantation, l'évaporation, l'extraction liquide-liquide, etc., réalisées seules ou en combinaison, est opérée de façon à récupérer au moins un flux liquide organique 24 contenant au moins le DAP et/ou le API_{H} et un flux liquide aqueux 28 contenant au moins l'eau et les AL_{H}. Le flux 24 peut alors être avantageusement renvoyés à l'étape g) du procédé selon l'invention de façon à poursuivre les réactions chimiques conduisant à l'acide phtalique et à améliorer ainsi le rendement en acide phtalique. Le flux 28 peut, quant à lui, être envoyé à une deuxième étape **h₂)** de séparation permettant de récupérer au moins deux flux liquides 23 et 25 contenant respectivement l'eau et les AL_{H}, l'étape h₂) pouvant être une séparation liquide-liquide ou gaz-liquide, et pouvant par exemple comprendre une séparation par distillation éventuellement suivie d'une séparation plus poussée, par exemple par membrane. Les flux 23 et 25 peuvent alors être avantageusement renvoyés respectivement à l'étape g) et à l'étape b) du procédé selon l'invention, de façon à alimenter ces étapes en eau ou solvant si ce dernier comprend un AL_{H} produit (par exemple pour le cas spécifique ou n = 1, l'AL_{H} produit est le méthanol), et ainsi optimiser les apports en réactifs/solvants du procédé. Dans le cas spécifique ou n = 1, l'AL_{H} produit étant le méthanol, ce dernier peut être également être extrait directement dans l'étape g), par exemple par un séparation gaz-liquide comme une distillation réactive, de façon à valoriser la formation de l'acide phtalique par déplacement chimique de l'équilibre thermodynamique de la réaction d'hydrolyse. La mise en œuvre des étapes h₁) et h₂) reste, dans ce cas, inchangée.

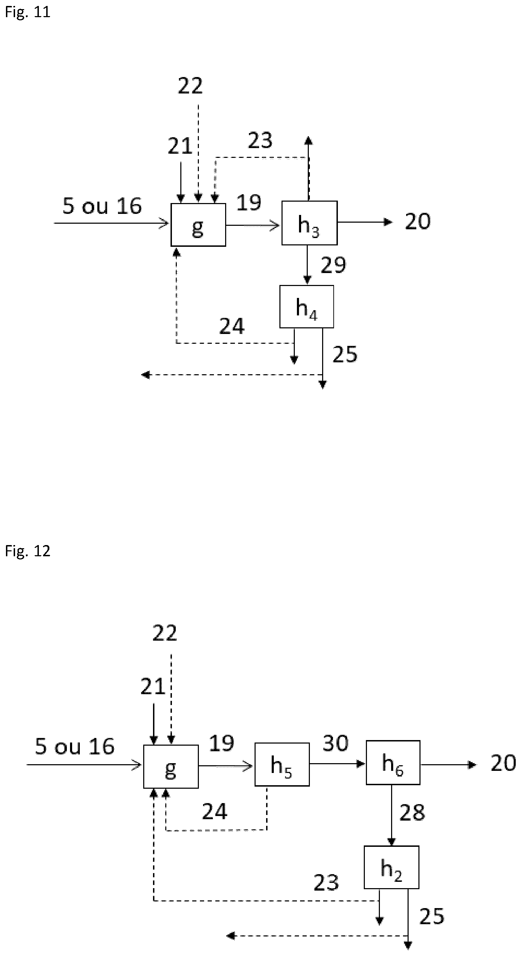

Un deuxième exemple de mise en œuvre de l'étape h) est représenté à la figure 11, et est particulièrement bien adapté lorsque que le DAP envoyé à l'étape g) du procédé selon l'invention à pour formule chimique C₆H₄(COOCₙH₂ₙ₊₁)₂ avec n > 8. Dans cette configuration, l'effluent 19 comporte au moins deux phases liquides immiscibles : une phase liquide aqueuse comprenant l'acide phtalique et une phase liquide organique contenant au moins l'API_{H} et/ou le DAP et/ou l'AL_{H}. L'effluent 19 est envoyé à une première étape **h₃)** combinant un changement de phase liquide-solide de l'acide phtalique et la séparation solide-liquide du milieu ainsi obtenu pour l'obtention d'au moins un flux solide comprenant l'acide phtalique (à l'état solide) 20, au moins un flux liquide organique 29 contenant au moins le DAP, l'API_{H} et l'AL_{H} et au moins un flux liquide aqueux 23 contenant au moins l'eau. Le changement de phase liquide-solide de l'acide phtalique et la séparation des flux ainsi obtenus peuvent avantageusement être mis en œuvre selon les mêmes techniques que celles décrites en relation avec la figure 10 ci-dessus pour l'étape h₁). Le flux 29 peut alors être envoyé à une deuxième étape **h₄)** de séparation permettant de récupérer au moins deux flux organiques liquides 24 et 25, le flux 24 contenant le DAP et/ou l'API_{H}, et le flux 25 comportant au moins les AL_{H}. Pour exemple, les techniques opérées pour la séparation de flux liquides des étapes h₃) et h₄) sont des extractions liquide-liquide. Comme dans le ou les modes de réalisation décrits en relation avec la figure 10, les divers flux 23, 24 et 25 peuvent également être avantageusement renvoyés à certaines étapes du procédé plus en amont. Par exemple le flux 23 comportant de l'eau résiduelle et le flux 24 comportant du DAP et l'API_{H} peuvent être recyclé à l'étape g), notamment pour alimenter en eau et/ou poursuivre les réactions chimiques conduisant respectivement à l'acide phtalique et ainsi améliorer son rendement, et le flux 25 comportant l'AL_{H} peut être recyclé à l'étape b), notamment pour alimenter en solvant cette étape b) si ledit solvant comprend un AL_{H} produit à l'étape g), et ainsi optimiser l'apport en solvant du procédé.

Un troisième exemple de mise en œuvre de l'étape h) est représenté à la figure 12, et est particulièrement bien adapté lorsque que le DAP envoyé à l'étape g) a pour formule chimique C₆H₄(COOCₙH₂ₙ₊₁)₂ avec n = 3. Selon cette configuration, l'effluent 19 comporte au moins deux phases liquides immiscibles : une phase liquide organique contenant au moins le DAP et/ou l'API_{H} et au moins une phase liquide aqueuse contenant au moins l'acide phtalique, l'AL_{H} et l'eau. L'effluent 19 est envoyé à une première étape de séparation **h₅)** pour séparer la phase liquide organique de la phase liquide aqueuse, de façon à produire séparément deux flux 24 et 30 comportant respectivement la phase organique et la phase aqueuse. Le flux 24 comportant la phase organique contenant le DAP et/ou l'API_{H} peut alors être avantageusement renvoyé à l'étape g) de façon à poursuivre les réactions chimiques conduisant à l'acide phtalique et à améliorer ainsi le rendement en ce produit. Le flux liquide aqueux 30 contenant au moins l'acide phtalique, l'AL_{H} et l'eau résiduelle est envoyé à une étape **h₆)** combinant un changement de phase liquide-solide de l'acide phtalique et une séparation solide-liquide, comme déjà décrit pour une partie de l'étape h₁) en relation avec la figure 10 (partie sur le changement d'état de l'acide phtalique de dissous à solide et séparation solide-liquide), pour produire au moins un flux solide d'acide phtalique 20, et un flux liquide aqueux 28. Ledit flux est envoyé à une étape **h₂)** telle que décrite en relation avec la figure 10, produisant deux les flux 23 et 25, pouvant être recyclés comme également déjà décrits en relation avec la figure 10.

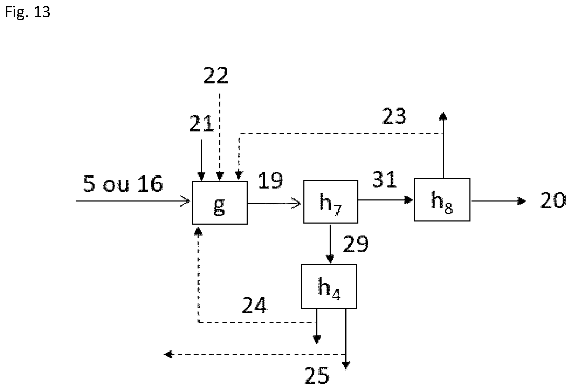

Un quatrième exemple de mise en œuvre de l'étape h) est représenté à la figure 13, et est particulièrement bien adapté lorsque que le DAP envoyé à l'étape g) a pour formule chimique C₆H₄(COOCₙH₂ₙ₊₁)₂ avec n > 8. Selon cette configuration, l'effluent 19 comprend au moins deux phases liquides immiscibles : une phase liquide organique contenant au moins le DAP, l'API_{H} et l'AL_{H} et au moins une phase liquide aqueuse contenant au moins l'acide phtalique et l'eau résiduelle. L'effluent 19 est envoyé à une première étape de séparation **h₇)** pour séparer la phase liquide organique de la phase liquide aqueuse, de façon à produire séparément les deux flux 29 et 31 comportant respectivement la phase organique et la phase aqueuse. Le flux liquide aqueux 31 contenant au moins l'acide phtalique et l'eau résiduelle est envoyé à une étape **h₈)** combinant un changement de phase liquide-solide de l'acide phtalique et une séparation solide-liquide, ainsi qu'une séparation comme déjà décrit pour une partie de l'étape h₁) en relation avec la figure 10 (partie sur le changement d'état de l'acide phtalique de dissous à solide et séparation solide-liquide), pour produire au moins un flux solide comprenant l'acide phtalique 20 et d'au moins un flux liquide aqueux 23 contenant l'eau résiduelle. Comme décrit précédemment, le flux liquide aqueux 23 produit à l'étape h₈) peut être avantageusement renvoyé à certaines étapes plus amont du procédé, par exemple à l'étape g). Le flux 29 produit à l'étape h₇) est envoyé à une étape **h₄)** tel que précédemment décrite en relation avec la figure 11, pour produire des flux 24 et 25, pouvant être recyclés comme également déjà décrits en relation avec la figure 11.

Il est possible de produire de l'anhydride phtalique en envoyant le flux solide d'AP 20 à une étape de déshydratation, l'anhydride phtalique pouvant être le composé de départ pris pour synthétiser les phtalates du PVC. La déshydratation de l'AP pour former de l'anhydride phtalique est connue, et une telle étape déshydratation peut être réalisée comme cela est par exemple décrit dans le brevet US3720692

### Procédé de recyclage

La présente invention porte également sur un procédé de recyclage d'un objet à base de PVC contenant au moins un phtalate, ledit procédé de recyclage comportant :
- le conditionnement de l'objet à base de PVC comprenant au moins un broyage ou un déchiquetage de l'objet à base de PVC pour former une charge de PVC sous forme de particules ;
- la récupération d'acide phtalique et d'un plastique PVC cible réutilisable à partir de ladite charge de PVC sous forme de particules selon le procédé le premier aspect de l'invention décrit plus haut en détail.

L'étape de conditionnement de l'objet à base de PVC peut inclure les différentes étapes détaillées plus haut pour le conditionnement préalable de la charge de PVC avant son introduction à l'étape a). Il est avantageux, dans une optique d'économie circulaire, d'utiliser l'acide phtalique obtenu par le procédé de récupération décrit pour obtenir à nouveau des phtalates adaptés à la formulation de plastiques PVC souples et/ou d'utiliser le plastique PVC cible produit par le procédé de récupération selon l'invention pour fabriquer un nouvel objet à base de PVC souple. Un tel objet peut alors être fabriqué plus facilement de manière à répondre aux normes en vigueur en ce qui concerne le phtalates, et inclure seulement des phtalates REACH compatibles, en étant fabriqué à partir de matières premières respectant ou adaptées pour respecter lesdites normes, i.e. le plastique PVC cible récupéré exempt de phtalates non REACH compatibles, et l'AP permettant la production de phtalates REACH compatibles.

### Procédé de fabrication

La présente invention concerne également un procédé de fabrication d'un objet à base de PVC souple comportant un plastique PVC recyclé et/ou un phtalate fabriqué à partir d'acide phtalique récupéré par le procédé selon le premier aspect de l'invention.

La présente invention porte également sur un procédé de fabrication d'un objet à base de PVC souple comportant un plastique PVC recyclé obtenu par le procédé de récupération d'acide phtalique et d'un plastique PVC cible réutilisable selon le premier aspect de l'invention décrit plus haut en détail.

Un tel procédé de fabrication comprend typiquement une étape de récupération d'acide phtalique et d'un plastique PVC cible réutilisable à partir d'une charge de PVC, comme détaillé plus haut, puis une étape de mélange dudit plastique PVC cible réutilisable avec des additifs, puis une étape de mise en forme dudit mélange.

### Exemple

Cet exemple illustre l'invention sans en limiter la portée, et illustre notamment l'extraction d'un phtalate contenu dans un plastique PVC, la conversion du phtalate en diméthylphtalate (DMP) en présence d'un catalyseur par méthanolyse, et la conversion du DMP en acide phtalique en présence d'un catalyseur et d'eau.

18,2 g d'une charge de plastique PVC (issue d'objets à base de PVC de type « tube médical »), sous la forme d'extrudés de taille moyenne de 2 mm, contenant 4,4 g de di-décyl phtalate (DIDP), sont introduits dans un réacteur agité par un système d'agitation mécanique, de type pales. 26,5 g de méthanol et 17,7 g de propanoate de méthyle (co-solvant organique) sont alors ajoutés, le ratio massique propanoate de méthyle/méthanol étant de 0,66 et le ratio molaire méthanol/DIDP de 84. 0,17 g de catalyseur (NaOMe) sont alors ajoutés au mélange précédent de telle sorte que le pourcentage massique NaOMe/DIDP soit de 4%.

Le réacteur est fermé de façon étanche, purgé à l'azote puis chauffé jusqu'à 100°C avec une pression autogène de l'ordre de 1,2 MPa et maintenu dans ces conditions pendant 4 h sous une agitation de 1000 tour/min. Le réacteur est ensuite refroidi.

Après 4 h, on obtient un solide et un liquide qui sont analysés.

Les analyses par chromatographie en phase gazeuse avec détection à ionisation de flamme (GC-FID) de la phase liquide montrent qu'elle contient 1,89 g de diméthylphtalate (DMP) issu de la conversion du DIDP et 0,05 g de decylmethylphtalate dû à une conversion partielle du DIDP. Le liquide contient également 3,11 g de décanol (C₁₀H₂₂O) issu de la réaction de méthanolyse du DIDP. L'identification a été rendu possible par la comparaison des temps de rétention de standards analytiques purs et la quantification a été réalisée à partir de la détermination des coefficients de réponse issus de l'analyse de ces mêmes standards.

Le solide obtenu a été préfractionné par chromatographie d'exclusion stérique SEC préparative équipée d'une double détection optique (UV/Visible) et réfractométrie (RI). Les fractions issues de la collecte ont été analysées par chromatographie liquide haute performance (HPLC) équipée d'une détection optique de type UV-Visible à visée quantitative. Les résultats indiquent la présence de DIDP dans le plastique PVC cible a une teneur inférieure à 1000 ppm, ce qui est conforme à réglementation européenne en vigueur.

Ces résultats montrent qu'on obtient un PVC exempt de phtalate conformément à l'invention, et que le DIDP a été converti à 99,9%. Dans cet exemple, l'extraction du DIDP et sa conversion sont réalisées dans une même étape.

A l'issue de cette méthanolyse les 1,89 g de DMP sont réintroduits dans un réacteur agité par un système d'agitation mécanique, de type pales. 52,56 g d'eau sont alors ajoutés, (ratio molaire eau/DMP de 300). 0,06 g de catalyseur qui est de l'acide p-toluène sulfonique (APTS) sont alors ajoutés au mélange précédent de sorte que le ratio massique APTS/DMP soit de 3%.

Le réacteur est fermé de façon étanche, purgé à l'azote puis chauffé jusqu'à 100°C avec une pression autogène de l'ordre de 1,2 MPa et maintenu dans ces conditions pendant 4 h sous une agitation de 1000 tour/min. Le réacteur est ensuite refroidi à 65°C.

Après 4 h, on le milieu réactionnel est refroidi à 10°C, ce qui conduit à la précipitation d'un solide majoritairement constitué d'acide phtalique. Le solide obtenu est filtré. Le liquide restant (filtrat) est alors de nouveau laissé à réagir par trois fois dans les mêmes conditions que précédemment décrites. A l'issue de chaque étape réactive, une étape de reprécipitation à froid permet d'extraire une phase solide majoritairement constituée d'acide phtalique. A l'issue de ce protocole les fractions solides secondaires sont réunies et analysées.

Les analyses par chromatographie en phase gazeuse avec détection à ionisation de flamme (GC-FID) de la phase solide montrent qu'elle contient 1,45 g de diacide phtalique (AP) issu de la conversion du DMP et 0,18 g de monométhyl phtalate (acide 2-(méthoxycarbonyl)benzoique) résultant d'une hydrolyse partielle du DMP. Le liquide quant à lui contient également 0,59 g de méthanol (C₁H₄O) issu de la réaction d'hydrolyse du DMP. L'identification a été rendue possible par la comparaison des temps de rétention de standards analytiques purs et la quantification a été réalisée à partir de la détermination des coefficients de réponse issus de l'analyse de ces mêmes standards.

Ces résultats montrent donc également que le DMP a été converti à 99,9%.

## Revendications

1. Procédé de récupération d'acide phtalique et d'un plastique PVC cible réutilisable à partir d'une charge de PVC contenant au moins un phtalate, comportant les étapes suivantes :
a) une extraction solide-liquide de ladite charge de PVC sous forme de particules (1) par mise en contact desdites particules de la charge de PVC avec un solvant (9) comportant au moins un alcool de formule CₙH₂ₙ₊₁OH, n entier positif inférieur à 4 ou supérieur à 8, pour produire une phase liquide enrichie en ledit phtalate et une phase solide comportant du plastique PVC appauvri en ledit phtalate ;
b) la transformation chimique dudit phtalate de ladite phase liquide en dialkylphtalate de formule C₆H₄(COOCₙH₂ₙ₊₁)₂ par transestérification au moyen dudit alcool pour enrichir ladite phase liquide en ledit dialkylphtalate ;
c) une séparation solide-liquide entre ladite phase solide et ladite phase liquide pour produire au moins un flux solide comportant le plastique PVC appauvri en ledit phtalate (6) afin de récupérer ledit plastique PVC cible ;
d) une séparation de ladite phase liquide, pour produire au moins un premier effluent liquide comportant ledit dialkylphtalate (5, 14) et un deuxième effluent liquide comprenant au moins ledit solvant (7, 12) ;
e) une purification optionnelle dudit premier effluent liquide (14) obtenu à l'étape d) comprenant ledit dialkylphtalate, du phtalate partiellement converti et/ou non converti à l'étape b) et éventuellement des impuretés solubles, pour produire un produit liquide (16) consistant essentiellement en ledit dialkylphtalate, et un résidu liquide (17) comprenant dudit phtalate partiellement converti et/ou non converti à l'étape b) et éventuellement lesdites impuretés solubles ;
f) une étape optionnelle supplémentaire f₁) et/ou étape optionnelle supplémentaire f₂) de transformation chimique par transestérification dudit phtalate non converti et/ou partiellement à l'étape b), en dialkylphtalate de formule C₆H₄(COOCₙH₂ₙ₊₁)₂ au moyen dudit alcool, ladite étape f₁) étant réalisée entre les étapes c) et d) par envoi de ladite phase liquide obtenue à l'issue de l'ensemble des étapes a), b) et c) dans un premier réacteur de transestérification supplémentaire pour produire un deuxième flux liquide (13) enrichi en ledit dialkylphtalate de formule C₆H₄(COOCₙH₂ₙ₊₁)₂, ledit deuxième flux liquide (13) étant envoyé à l'étape d), et ladite étape f₂) étant réalisée successivement à l'étape e) par envoi dudit résidu liquide (17) dans un deuxième réacteur de transestérification supplémentaire pour produire un troisième flux liquide (15) enrichi en ledit dialkylphtalate de formule C₆H₄(COOCₙH₂ₙ₊₁)₂, ledit troisième flux liquide (15) étant renvoyé à l'étape d) ;
g) une transformation chimique dudit dialkylphtalate obtenu à l'étape d) ou à l'étape optionnelle e) en acide phtalique de formule C₆H₄(COOH)₂ par hydrolyse au moyen d'eau pour produire un effluent (19) comportant une phase aqueuse comprenant ledit acide phtalique ;
h) une séparation dudit acide phtalique issu de l'étape g) et mis sous une forme solide pour produire au moins un flux solide d'acide phtalique (20).

2. Procédé selon la revendication 1, dans lequel les étapes a) et b) sont mises en œuvre au sein d'une même opération unitaire.

3. Procédé selon la revendication 1, dans lequel les étapes a) et b) font l'objet de deux opérations unitaires distinctes, l'étape a) produisant un flux (2) comportant ladite phase liquide et ladite phase solide envoyé à l'étape c) de séparation solide-liquide réalisée entre les étapes a) et b), l'étape c) produisant ledit flux comportant le plastique PVC appauvri en ledit phtalate (6) et un premier flux liquide (18) comportant ladite phase liquide envoyée à l'étape b).

4. Procédé selon l'une quelconque des revendications précédentes, dans lequel l'hydrolyse à l'étape g) est réalisée en présence d'un catalyseur d'hydrolyse acide, de préférence un catalyseur homogène acide choisi dans la liste constituée par les catalyseurs acides de Brônsted minéraux, de préférence l'acide chlorhydrique, l'acide sulfurique, l'acide phosphorique, les catalyseurs acides de Brônsted organiques, de préférence l'acide *p*-toluènesulfonique, et les catalyseurs acides de Lewis, de préférence AlF₃, ou un catalyseur hétérogène acide choisi dans la liste constituée par les alumines, les alumines chlorées, les alumines fluorées, les aluminosilicates mésoporeux, les zéolithes et leurs mélanges avec d'autres oxydes, les résines échangeuses d'ions (H+), de préférence les résines sulfoniques.

5. Procédé selon l'une quelconque des revendications précédentes, dans lequel l'hydrolyse à l'étape g) est réalisée à une température comprise entre la température ambiante et 150°C, de préférence comprise entre 40°C et 130°C, à une pression comprise entre la pression atmosphérique et 5,0 MPa, de préférence comprise entre la pression atmosphérique et 2,0 MPa, et pendant une durée comprise entre 1 minute et 10 heures, de préférence comprise entre 10 minutes et 4 heures.

6. Procédé selon l'une quelconque des revendications précédentes, dans lequel l'hydrolyse à l'étape g) est réalisée de sorte que le rapport molaire entre la quantité d'eau et la quantité dudit au moins un phtalate à transformer extrait à l'étape a) soit compris entre 100 et 9000.

7. Procédé selon l'une quelconque des revendications précédentes, dans lequel l'étape h) comporte changement de phase de l'acide phtalique de l'état dissous dans ladite phase aqueuse à un état solide et une séparation solide-liquide pour produire ledit flux solide d'acide phtalique (20) et au moins un flux liquide aqueux (23, 28).

8. Procédé selon l'une quelconque des revendications précédentes, dans lequel ledit premier effluent liquide (5) à l'étape d) ou ledit produit liquide (16) à l'étape optionnelle e) consiste essentiellement en ledit dialkylphtalate.

9. Procédé selon l'une quelconque des revendications précédentes, dans lequel ledit flux solide comportant le plastique PVC appauvri en phtalates (6) est recyclé au moins en partie à l'étape a).

10. Procédé selon l'une quelconque des revendications précédentes, dans lequel ledit alcool est choisi dans la liste constituée par le méthanol, l'éthanol, le *n*-propanol, l'*i*-propanol, et de préférence le méthanol, ou dans la liste constituée par le nonanol, linéaire ou ramifié, le décanol, linéaire ou ramifié, le undécanol, linéaire ou ramifié, le dodécanol, linéaire ou ramifié, et de préférence le nonanol ou le décanol.

11. Procédé selon l'une quelconque des revendications précédentes, dans lequel ledit solvant comprend en outre un co-solvant organique, de préférence ledit co-solvant organique étant choisi parmi un ester dérivé dudit alcool et étant de formule R'COOCₙH₂ₙ+1, R' étant un groupement alkyle, de préférence comprenant entre 1 et 3 atomes de carbone, et un éther, de préférence ledit co-solvant organique est choisi dans le groupe constitué par l'acétate de méthyle, le propanoate de méthyle, et le cyclopentylméthyléther, et ledit co-solvant organique étant additionné audit alcool de sorte que le ratio massique entre ledit co-solvant organique et ledit alcool soit compris entre 0,01 et 4.

12. Procédé selon l'une quelconque des revendications précédentes, dans lequel la transformation chimique réalisée par transestérification à l'étape b), et éventuellement à l'étape f₁) et/ou f₂), est opérée :
- à une température comprise entre la température ambiante et 200°C, de préférence comprise entre 40°C et 180°C,
à une pression comprise entre la pression atmosphérique et 11,0 MPa, de préférence comprise entre la pression atmosphérique et 5,0 MPa,
- pendant une durée comprise entre 1 minute et 10 heures, de préférence comprise entre 10 minutes et 4 heures,
- avec un rapport molaire entre la quantité dudit alcool du solvant (9) et la quantité dudit phtalate à extraire ou à transformer est compris entre 2 et 250, de préférence compris entre 4 et 90, et
- en présence d'un catalyseur de transestérification, de préférence choisi dans la liste constituée par les catalyseurs homogènes basiques, ou acides de Brônsted minéraux ou organiques, ou acides de Lewis, et les catalyseurs hétérogènes formés par des oxydes de métaux alcalino-terreux, ou des carbonates ou hydrogéno-carbonates de métaux alcalins et/ou alcalino-terreux, ou des métaux alcalins supportés sur alumines ou zéolites, ou des oxydes de zinc et leurs mélanges avec d'autres oxydes, ou des résines échangeuses d'ions.

13. Procédé selon l'une quelconque des revendications précédentes, dans lequel ledit au moins un phtalate de ladite charge de PVC est un phtalate de formule brute C₆H₄(COOR₁)(COOR₂) dont les groupements esters sont en position ortho du noyau benzénique, R₁ ou R₂ étant choisis indépendamment parmi l'un des éléments du groupe constitué par une chaîne alkyle, linéaire ou ramifiée ou cyclique, une chaîne alkoxyalkyle, linéaire ou ramifiée, ou une chaîne aryle ou alkylaryle, R₁ et/ou R₂ comprenant de préférence entre 1 et 20 atomes de carbone, voire entre 1 et 15 atomes de carbone.

14. Procédé selon l'une quelconque des revendications précédentes, dans lequel ledit plastique PVC cible est sensiblement exempt dudit phtalate, et de préférence comprend moins de 0,1% massique au total de phtalates choisis dans la liste constituée par le phtalate de dibutyle, phtalate de dioctyle ou de diéthylhexyl, phtalate de benzyle et butyle, phtalate de dibutyle, phtalate de diisobutyle, phtalate de dipentyle, phtalate de diisopentyle, phtalate de n-pentyle et isopentyle, phtalate de dihexyle, phtalate de bis(2-méthoxyéthyle), et leurs mélanges.

15. Procédé de recyclage d'un objet à base de PVC contenant au moins un phtalate comportant :
- le conditionnement dudit objet à base de PVC comprenant au moins un broyage ou un déchiquetage dudit objet à base de PVC pour former une charge de PVC sous forme de particules ;
- la récupération d'acide phtalique et d'un plastique PVC cible réutilisable à partir de ladite charge de PVC sous forme de particules selon l'une quelconque des revendications 1 à 14.

## Patentansprüche

1. Verfahren zur Rückgewinnung von Phthalsäure und eines wiederverwendbaren Ziel-PVC-Kunststoffs ausgehend von einem PVC-Einsatzmaterial, das mindestens ein Phthalat enthält, umfassend die folgenden Schritte:
a) eine Fest-Flüssig-Extraktion des PVC-Einsatzmaterials in Form von Partikeln (1) durch Inkontaktbringen der Partikel des PVC-Einsatzmaterials mit einem Solvens (9), das mindestens einen Alkohol mit der Formel CₙH₂ₙ₊₁OH enthält, wobei n ist eine positive ganze Zahl kleiner als 4 oder größer als 8 ist, um eine an dem Phthalat angereicherte flüssige Phase und eine feste Phase, die an Phthalat abgereicherten PVC-Kunststoff umfasst, zu erzeugen;
b) die chemische Umwandlung des Phthalats der flüssigen Phase in ein Dialkylphthalat mit der Formel C₆H₄(COOCₙH₂ₙ₊₁)₂ durch Umesterung mittels des Alkohols, um die flüssige Phase an dem Dialkylphthalat anzureichern;
c) eine Fest-Flüssig-Trennung zwischen der festen Phase und der flüssigen Phase, um mindestens einen festen Strom (6) zu erzeugen, der den an dem Phthalat abgereicherten PVC-Kunststoff umfasst, um den Ziel-PVC-Kunststoff zurückzugewinnen;
d) eine Trennung der flüssigen Phase, um mindestens einen ersten flüssigen Abstrom (5, 14), der das Dialkylphthalat umfasst, und einen zweiten flüssigen Abstrom (7, 12), der mindestens das Solvens umfasst, zu erzeugen;
e) eine optionale Reinigung des im Schritt d) erhaltenen ersten flüssigen Abstroms (14), der das Dialkylphthalat umfasst, von dem im Schritt b) teilweise umgewandelten und/oder nicht umgewandelten Phthalat und gegebenenfalls von den löslichen Verunreinigungen, um ein flüssiges Produkt (16), das im Wesentlichen aus dem Dialkylphthalat besteht, und einen flüssigen Rückstand (17), der das im Schritt b) teilweise umgewandelte und/oder nicht umgewandelte Phthalat und gegebenenfalls die löslichen Verunreinigungen umfasst, zu erzeugen;
f) einen zusätzlichen optionalen Schritt f₁) und/oder zusätzlichen optionalen Schritt f₂) der chemischen Umwandlung durch Umesterung des im Schritt b) nicht und/oder teilweise umgewandelten Phthalats in Dialkylphthalat mit der Formel C₆H₄(COOCₙH₂ₙ₊₁)₂ mittels des Alkohols, wobei der Schritt f₁) zwischen den Schritten c) und d) durch Leiten der am Ende sämtlicher Schritte a), b) und c) erhaltenen flüssigen Phase in einen ersten zusätzlichen Umesterungsreaktor ausgeführt wird, um einen zweiten flüssigen Strom (13) zu erzeugen, der an dem Dialkylphthalat mit der Formel C₆H₄(COOCₙH₂ₙ₊₁)₂ angereichert ist, wobei der zweite flüssige Strom (13) zu dem Schritt d) geleitet wird, und wobei der Schritt f₂) nach dem Schritt e) durch Leiten des flüssigen Rückstands (17) in einen zweiten zusätzlichen Umesterungsreaktor ausgeführt wird, um einen dritten flüssigen Strom (15) zu erzeugen, der an dem Dialkylphthalat mit der Formel C₆H₄(COOCₙH₂ₙ₊₁)₂ angereichert ist, wobei der dritte flüssige Strom (15) zu dem Schritt d) zurückgeleitet wird;
g) eine chemische Umwandlung des im Schritt d) oder im optionalen Schritt e) erhaltenen Dialkylphthalats zu Phthalsäure mit der Formel C₆H₄(COOH)₂ durch Hydrolyse mittels Wasser, um einen Abstrom (19) zu erzeugen, der eine wässrige Phase umfasst, die die Phthalsäure umfasst;
h) eine Trennung der aus dem Schritt g) hervorgegangenen und in eine feste Form gebrachten Phthalsäure, um mindestens einen festen Phthalsäurestrom (20) zu erzeugen.

2. Verfahren nach Anspruch 1, bei dem die Schritte a) und b) innerhalb derselben Grundoperation durchgeführt werden.

3. Verfahren nach Anspruch 1, bei dem die Schritte a) und b) Gegenstand von zwei verschiedenen Grundoperationen sind, wobei der Schritt a) einen die flüssige Phase und die feste Phase umfassenden Strom (2) erzeugt, der zu dem zwischen den Schritten a) und b) ausgeführten Schritt c) der Fest-Flüssig-Trennung geleitet wird, wobei der Schritt c) den Strom (6), der den an dem Phthalat abgereicherten PVC-Kunststoff umfasst, und einen ersten flüssigen Strom (18), der die zu dem Schritt b) geleitete flüssige Phase umfasst, erzeugt.

4. Verfahren nach einem der vorhergehenden Ansprüche, bei dem die Hydrolyse im Schritt g) in Gegenwart eines sauren Hydrolysekatalysators ausgeführt wird, vorzugsweise eines sauren homogenen Katalysators, der aus der Liste bestehend aus den mineralischen sauren Brönsted-Katalysatoren, vorzugsweise Salzsäure, Schwefelsäure, Phosphorsäure, den organischen sauren Brönsted-Katalysatoren, vorzugsweise *p*-Toluolsulfonsäure, und den sauren Lewis-Katalysatoren, vorzugsweise AlF₃, gewählt ist, oder eines sauren heterogenen Katalysators, der aus der Liste bestehend aus Aluminen, chlorierten Aluminen, fluorierten Aluminen, mesoporösen Aluminosilikaten, Zeolithen und deren Mischungen mit anderen Oxiden, Ionenaustauscherharzen (H+), vorzugsweise Sulfonharzen, gewählt ist.

5. Verfahren nach einem der vorhergehenden Ansprüche, bei dem die Hydrolyse im Schritt g) bei einer Temperatur zwischen der Umgebungstemperatur und 150 °C, vorzugsweise zwischen 40 °C und 130 °C, bei einem Druck zwischen dem atmosphärischem Druck und 5,0 MPa, vorzugsweise zwischen dem atmosphärischem Druck und 2,0 MPa, und während einer Dauer zwischen 1 Minute und 10 Stunden, vorzugsweise zwischen 10 Minuten und 4 Stunden, ausgeführt wird.

6. Verfahren nach einem der vorhergehenden Ansprüche, bei dem die Hydrolyse im Schritt g) so ausgeführt wird, dass das molare Verhältnis zwischen der Wassermenge und der Menge des im Schritt a) extrahierten, mindestens einen umzuwandelnden Phthalats zwischen 100 und 9000 beträgt.

7. Verfahren nach einem der vorhergehenden Ansprüche, bei dem der Schritt h) eine Phasenänderung der Phthalsäure von dem in der wässrigen Phase gelösten Zustand zu einem festen Zustand und eine Fest-Flüssig-Trennung umfasst, um den festen Phthalsäurestrom (20) und mindestens einen wässrigen flüssigen Strom (23, 28) zu erzeugen.

8. Verfahren nach einem der vorhergehenden Ansprüche, bei dem der erste flüssige Abstrom (5) im Schritt d) oder das flüssige Produkt (16) im optionalem Schritt e) im Wesentlichen aus dem Dialkylphthalat besteht.

9. Verfahren nach einem der vorhergehenden Ansprüche, bei dem der feste Strom (6), der den an Phthalaten abgereicherten PVC-Kunststoff umfasst, mindestens teilweise zum Schritt a) zurückgeführt wird.

10. Verfahren nach einem der vorhergehenden Ansprüche, bei dem der Alkohol aus der Liste bestehend aus Methanol, Ethanol, *n*-Propanol, *i*-Propanol und vorzugsweise Methanol oder aus der Liste bestehend aus geradkettigem oder verzweigtem Nonanol, geradkettigem oder verzweigtem Decanol, geradkettigem oder verzweigtem Undecanol, geradkettigem oder verzweigtem Dodecanol und vorzugsweise Nonanol oder Decanol gewählt ist.

11. Verfahren nach einem der vorhergehenden Ansprüche, bei dem das Solvens ferner ein organisches Co-Solvent umfasst, wobei das organische Co-Solvens vorzugsweise aus einem Ester, der von dem Alkohol abgeleitet ist und die Formel R'COOCₙH₂ₙ+1 aufweist, wobei R' eine Alkylgruppe mit vorzugsweise 1 bis 3 Kohlenstoffatomen ist, und einem Ether gewählt ist, wobei das organische Co-Solvens vorzugsweise aus der Gruppe bestehend aus Methylacetat, Methylpropanoat und Cyclopentylmethylether gewählt ist und wobei das organische Co-Solvens dem Alkohol so zugegeben wird, dass das Massenverhältnis zwischen dem organischen Co-Solvens und dem Alkohol zwischen 0,01 und 4 beträgt.

12. Verfahren nach einem der vorhergehenden Ansprüche, bei dem die chemische Umwandlung, die durch Umesterung im Schritt b) und gegebenenfalls im Schritt f₁) und/oder f₂) ausgeführt wird, vorgenommen wird:
- bei einer Temperatur zwischen der Umgebungstemperatur und 200 °C, vorzugsweise zwischen 40 °C und 180 °C,
bei einem Druck zwischen dem atmosphärischen Druck und 11,0 MPa, vorzugsweise zwischen dem atmosphärischen Druck und 5,0 MPa,
- während einer Dauer zwischen 1 Minute und 10 Stunden, vorzugsweise zwischen 10 Minuten und 4 Stunden,
- mit einem molaren Verhältnis zwischen der Menge des Alkohols des Solvens (9) und der Menge des zu extrahierenden oder umzuwandelnden Phthalats zwischen 2 und 250, vorzugsweise zwischen 4 und 90, und
- in Gegenwart eines Umesterungskatalysators, der vorzugsweise aus der Liste bestehend aus den basischen homogenen Katalysatoren oder mineralischen oder organischen Brönsted-Säuren oder Lewis-Säuren und den heterogenen Katalysatoren, die durch Erdalkalimetalloxide oder Carbonate oder Hydrogencarbonate von Alkali- und/oder Erdalkalimetallen oder auf Aluminiumoxiden oder Zeolithen geträgerte Alkalimetalle oder Zinkoxide und deren Mischungen mit anderen Oxiden oder Ionenaustauscherharze gebildet werden, gewählt ist.

13. Verfahren nach einem der vorhergehenden Ansprüche, bei dem das mindestens eine Phthalat des PVC-Einsatzmaterials ein Phthalat mit der Summenformel C₆H₄(COOR₁)(COOR₂) ist, dessen Estergruppen in Ortho-Position des Benzolrings sind, wobei R₁ oder R₂ unabhängig aus einem der Elemente der Gruppe bestehend aus einer geradkettigen oder verzweigten oder zyklischen Alkylkette, einer geradkettigen oder verzweigten Alkoxyalkylkette oder einer Aryl- oder Alkylarylkette gewählt sind, wobei R₁ und/oder R₂ vorzugsweise zwischen 1 und 20 Kohlenstoffatome oder sogar zwischen 1 und 15 Kohlenstoffatome umfassen.

14. Verfahren nach einem der vorhergehenden Ansprüche, bei dem der Ziel-PVC-Kunststoff im Wesentlichen frei von dem Phthalat ist und vorzugsweise insgesamt weniger als 0,1 Massen-% Phthalate umfasst, die aus der Liste bestehend aus Dibutylphthalat, Dioctyl- oder Diethylhexylphthalat, Benzylbutylphthalat, Dibutylphthalat, Diisobutylphthalat, Dipentylphthalat, Diisopentylphthalat, n-Pentyl-Isopentylphthalat, Dihexylphthalat, Bis(2-methoxyethyl)phthalat und Mischungen davon gewählt sind.

15. Verfahren zum Recyceln eines Gegenstands auf Basis von PVC, das mindestens ein Phthalat enthält, umfassend:
- das Aufbereiten des Gegenstands auf Basis von PVC, umfassend zumindest ein Mahlen oder Schreddern des Gegenstands auf Basis von PVC, um ein PVC-Einsatzmaterial in Form von Partikeln zu bilden;
- das Rückgewinnen von Phthalsäure und eines wiederverwendbaren Ziel-PVC-Kunststoffs ausgehend von dem PVC-Einsatzmaterial in Form von Partikeln nach einem der Ansprüche 1 bis 14.

## Claims

1. Process for recovering phthalic acid and a reusable target PVC plastic from a PVC feedstock containing at least one phthalate, including the following steps:
a) a solid-liquid extraction of said PVC feedstock in the form of particles (1) by placing said particles of the PVC feedstock in contact with a solvent (9) including at least one alcohol of formula CₙH₂ₙ₊₁OH, n being a positive integer less than 4 or greater than 8, to produce a liquid phase enriched in said phthalate and a solid phase including PVC plastic depleted in said phthalate;
b) chemical transformation of said phthalate of said liquid phase into dialkyl phthalate of formula C₆H₄(COOCₙH₂ₙ₊₁)₂ by transesterification using said alcohol to enrich said liquid phase in said dialkyl phthalate;
c) a solid-liquid separation between said solid phase and said liquid phase to produce at least one solid stream including the PVC plastic depleted in said phthalate (6) so as to recover said target PVC plastic;
d) a separation of said liquid phase, to produce at least a first liquid effluent including said dialkyl phthalate (5, 14) and a second liquid effluent comprising at least said solvent (7, 12);
e) an optional purification of said first liquid effluent (14) obtained in step d) comprising said dialkyl phthalate, from the phthalate partially converted and/or not converted in step b) and optionally from the soluble impurities, to produce a liquid product (16) consisting essentially of said dialkyl phthalate, and a liquid residue (17) comprising said phthalate partially converted and/or not converted in step b) and optionally said soluble impurities.
f) an optional additional step f₁) and/or optional additional step f₂) of chemical transformation, by transesterification, of said phthalate not converted and/or partially converted in step b), to dialkyl phthalate of formula C₆H₄(COOCₙH₂ₙ₊₁)₂ using said alcohol, said step f₁) being carried out between steps c) and d) by sending said liquid phase obtained on conclusion of all of steps a), b) and c) to a first additional transesterification reactor to produce a second liquid stream (13) enriched in said dialkyl phthalate of formula C₆H₄(COOCₙH₂ₙ₊₁)₂, said second liquid stream (13) being sent to step d), and said step f₂) being carried out after step e) by sending said liquid residue (17) to a second additional transesterification reactor to produce a third liquid stream (15) enriched in said dialkyl phthalate of formula C₆H₄(COOCₙH₂ₙ₊₁)₂, said third liquid stream (15) being sent back to step d);
g) a chemical transformation of said dialkyl phthalate obtained in step d) or in the optional step e) to phthalic acid of formula C₆H₄(COOH)₂ by hydrolysis using water to produce an effluent (19) including an aqueous phase comprising said phthalic acid;
h) a separation of said phthalic acid from step g) and converted to a solid form in order to produce at least one solid stream of phthalic acid (20).

2. Process according to Claim 1, in which steps a) and b) are performed within the same individual operation.

3. Process according to Claim 1, in which steps a) and b) form the subject of two distinct individual operations, step a) producing a stream (2) comprising said liquid phase and said solid phase sent to the solid-liquid separation step c) carried out between steps a) and b), step c) producing said stream comprising the PVC plastic depleted in said phthalate (6) and a first liquid stream (18) comprising said liquid phase sent to step b).

4. Process according to any one of the preceding claims, in which the hydrolysis in step g) is carried out in the presence of an acid hydrolysis catalyst, preferably a homogeneous acid catalyst chosen from the list consisting of mineral Brønsted acid catalysts, preferably hydrochloric acid, sulfuric acid or phosphoric acid, organic Brønsted acid catalysts, preferably *p-*toluenesulfonic acid, and Lewis acid catalysts, preferably AlF₃, or a heterogeneous acid catalyst chosen from the list consisting of aluminas, chlorinated aluminas, fluorinated aluminas, mesoporous aluminosilicates, zeolites and mixtures thereof with other oxides, (H⁺) ion-exchange resins, preferably sulfonic resins.

5. Process according to any one of the preceding claims, in which the hydrolysis in step g) is carried out at a temperature of between room temperature and 150°C, preferably between 40°C and 130°C, at a pressure of between atmospheric pressure and 5.0 MPa, preferably between atmospheric pressure and 2.0 MPa, and for a time of between 1 minute and 10 hours, preferably between 10 minutes and 4 hours.

6. Process according to any one of the preceding claims, in which the hydrolysis in step g) is carried out so that the mole ratio of the amount of water to the amount of said at least one phthalate to be transformed which is extracted in step a) is between 100 and 9000.

7. Process according to any one of the preceding claims, in which step h) comprises a phase change of the phthalic acid from the dissolved state in said aqueous phase to a solid state and a solid-liquid separation to produce said solid stream of phthalic acid (20) and at least one aqueous liquid stream (23, 28).

8. Process according to any one of the preceding claims, in which said first liquid effluent (5) in step d) or said liquid product (16) in optional step e) consists essentially of said dialkyl phthalate.

9. Process according to any one of the preceding claims, in which said solid stream comprising the phthalate-depleted PVC plastic (6) is recycled at least in part to step a).

10. Process according to any one of the preceding claims, in which said alcohol is chosen from the list consisting of methanol, ethanol, n-propanol, i-propanol, and preferably methanol, or from the list consisting of linear or branched nonanol, linear or branched decanol, linear or branched undecanol, linear or branched dodecanol, and preferably nonanol or decanol.

11. Process according to any one of the preceding claims, in which said solvent also comprises an organic cosolvent, said organic cosolvent preferably being chosen from an ester derived from said alcohol and having the formula R'COOCₙH₂ₙ₊₁, R' being an alkyl group preferably comprising between 1 and 3 carbon atoms, and an ether, said organic cosolvent is preferably chosen from the group consisting of methyl acetate, methyl propanoate and cyclopentyl methyl ether, and said organic cosolvent being added to said alcohol so that the weight ratio of said organic cosolvent to said alcohol is between 0.01 and 4.

12. Process according to any one of the preceding claims, in which the chemical transformation carried out by transesterification in step b), and optionally in step f₁) and/or f₂), is carried out:
- at a temperature between room temperature and 200°C, preferably between 40°C and 180°C,
- at a pressure between atmospheric pressure and 11.0 MPa, preferably between atmospheric pressure and 5.0 MPa,
- for a time of between 1 minute and 10 hours, preferably between 10 minutes and 4 hours,
- with a mole ratio of the amount of said alcohol of the solvent (9) to the amount of said phthalate to be extracted or transformed is between 2 and 250, preferably between 4 and 90, and
- in the presence of a transesterification catalyst preferably chosen from the list consisting of homogeneous basic, or mineral or organic Brønsted acid or Lewis acid catalysts, and heterogeneous catalysts formed by alkaline-earth metal oxides, or alkali metal and/or alkaline-earth metal carbonates or hydrogen carbonates, or alkali metals supported on aluminas or zeolites, or zinc oxides and mixtures thereof with other oxides, or ion-exchange resins.

13. Process according to any one of the preceding claims, in which said at least one phthalate of said PVC feedstock is a phthalate of empirical formula C₆H₄(COOR₁)(COOR₂) in which the ester groups are in the ortho position of the benzene ring, R₁ or R₂ being chosen independently from one of the elements of the group consisting of a linear or branched or cyclic alkyl chain, a linear or branched alkoxyalkyl chain, or an aryl or alkylaryl chain, R₁ and/or R₂ preferably comprising between 1 and 20 carbon atoms, or even between 1 and 15 carbon atoms.

14. Process according to any one of the preceding claims, in which said target PVC plastic is substantially free of said phthalate, and preferably comprises less than 0.1% by weight in total of phthalates chosen from the list consisting of dibutyl phthalate, dioctyl phthalate or diethylhexyl phthalate, benzyl butyl phthalate, dibutyl phthalate, diisobutyl phthalate, dipentyl phthalate, diisopentyl phthalate, n-pentyl isopentyl phthalate, dihexyl phthalate, bis(2-methoxyethyl) phthalate, and mixtures thereof.

15. Process for recycling a PVC-based object containing at least one phthalate, including:
- the conditioning of said PVC-based object comprising at least milling or shredding of said PVC-based object to form a PVC feedstock in the form of particles;
- the recovery of phthalic acid and of a reusable target PVC plastic from said PVC feedstock in the form of particles according to any one of Claims 1 to 14.
